# EUROPEAN PATENT APPLICATION

(11) **EP 3 599 245 A1**
(43) Date of publication of application: **29.01.2020**
(21) Application number: 18186029.7
(22) Date of filing: 27.07.2018
(51) Int. Cl.: C07K 7/08, A61K 38/04

(54) **IL-17A BINDING POLYPEPTIDES AND MEDICAL USES THEREOF**

(71) Applicant: Dompé farmaceutici S.p.A., 20122 Milan (IT)
(72) Inventor: ALLEGRETTI, Marcello, 00179 Roma (RM) (IT); ARAMINI, Andrea, 67100 L'Aquila (AQ) (IT); BECCARI, Andrea, 67100 L'Aquila (AQ) (IT); GEMEI, Marica, 80126 Napoli (NA) (IT); MANTELLI, Flavio, 00141 Roma (RM) (IT)
(74) Representative: Pistolesi, Roberto

(57) **Abstract**

The invention relates to IL-17A binding polypeptides, inhibitor of the complex IL17A/IL17RA/ILRC interaction, and the corresponding bioconjugates, their pharmaceutical composition and the medical use thereof in the treatment of inflammatory and autoimmune ophthalmic diseases.

SEQUENCE LISTING

<110> Dompe

<120> IL-17 Peptide inhibitors

<160> 157

<170> BiSSAP 1.2

<210> 1
<211> 15
<212> PRT
<213> Homo sapiens

<400> 1
Ile His Val Thr Ile Pro Ala Asp Leu Trp Asp Trp Ile Asn Lys
1               5                   10                  15

<210> 2
<211> 15
<212> PRT
<213> Homo sapiens

<400> 2
Val His Val Thr Ile Pro Ala Asp Leu Trp Asp Trp Ile Asn Lys
1               5                   10                  15

<210> 3
<211> 15
<212> PRT
<213> Homo sapiens

<400> 3
Ile Met Val Thr Ile Pro Ala Asp Leu Trp Asp Trp Ile Asn Lys
1               5                   10                  15

<210> 4
<211> 15
<212> PRT
<213> Homo sapiens

<400> 4
Val Met Val Thr Ile Pro Ala Asp Leu Trp Asp Trp Ile Asn Lys
1               5                   10                  15

<210> 5
<211> 15
<212> PRT
<213> Homo sapiens

<400> 5
Ile His Val Thr Ile Pro Ala Glu Leu Trp Asp Trp Ile Asn Lys
1               5                   10                  15

<210> 6
<211> 15
<212> PRT

<213> Homo sapiens

<400> 6
Ile His Val Thr Ile Pro Ala Asp Leu Tyr Asp Trp Ile Asn Lys
1               5                   10                  15

<210> 7
<211> 15
<212> PRT
<213> Homo sapiens

<400> 7
Ile His Val Thr Ile Pro Ala Asp Leu Tyr Glu Trp Ile Asn Lys
1               5                   10                  15

<210> 8
<211> 15
<212> PRT
<213> Homo sapiens

<400> 8
Ile His Val Thr Ile Pro Ala Asp Leu Trp Asp Trp Val Asn Lys
1               5                   10                  15

<210> 9
<211> 15
<212> PRT
<213> Homo sapiens

<400> 9
Ile His Val Thr Ile Pro Ala Asp Leu Trp Asp Trp Ile Arg Lys
1               5                   10                  15

<210> 10
<211> 15
<212> PRT
<213> Homo sapiens

<400> 10
Ile His Val Thr Ile Pro Ala Asp Leu Trp Asp Trp Ile Arg Lys
1               5                   10                  15

<210> 11
<211> 15
<212> PRT
<213> Homo sapiens

<400> 11
Ile His Val Thr Ile Pro Ala Asp Leu Trp Asp Trp Ile Asn Arg
1               5                   10                  15

<210> 12
<211> 15
<212> PRT
<213> Homo sapiens

<400> 12
Ile His Val Thr Ile Pro Ala Asp Leu Trp Asp Trp Ile Glu Lys
1               5                   10                  15

<210> 13
<211> 15
<212> PRT
<213> Homo sapiens


<400> 13
Ile His Val Thr Ile Pro Ala Asp Leu Trp Asp Trp Ile Asn Glu
1               5                   10                  15

<210> 14
<211> 15
<212> PRT
<213> Homo sapiens


<400> 14
Ile His Val Thr Ile Pro Ala Asp Leu Tyr Glu Trp Ile Asn Lys
1               5                   10                  15

<210> 15
<211> 15
<212> PRT
<213> Homo sapiens


<400> 15
Ile His Val Thr Ile Pro Ala Asp Leu Trp Asp Trp Val Arg Arg
1               5                   10                  15

<210> 16
<211> 15
<212> PRT
<213> Homo sapiens


<400> 16
Ile His Val Thr Ile Pro Ala Asp Leu Trp Asp Trp Val Glu Glu
1               5                   10                  15

<210> 17
<211> 15
<212> PRT
<213> Homo sapiens


<400> 17
Val Met Val Thr Ile Pro Ala Asp Leu Tyr Glu Trp Ile Asn Lys
1               5                   10                  15

<210> 18
<211> 15
<212> PRT
<213> Homo sapiens


<400> 18
Val Met Val Thr Ile Pro Ala Asp Leu Tyr Glu Trp Ile Arg Arg
1               5                   10                  15

<210> 19
<211> 15
<212> PRT
<213> Homo sapiens

<400> 19
Val Met Val Thr Ile Pro Ala Asp Leu Tyr Glu Trp Ile Glu Glu
1               5                   10                  15

<210> 20
<211> 21
<212> PRT
<213> Homo sapiens

<400> 20
Val Met Val Thr Ile Pro Ala Asp Leu Tyr Glu Trp Ile Glu Glu Arg
1               5                   10                  15
Arg Arg Arg Arg Arg
            20

<210> 21
<211> 18
<212> PRT
<213> Homo sapiens

<400> 21
Val Met Val Thr Ile Pro Ala Asp Leu Tyr Glu Trp Ile Glu Glu Arg
1               5                   10                  15
Arg Arg

<210> 22
<211> 23
<212> PRT
<213> Homo sapiens

<400> 22
Val Met Val Thr Ile Pro Ala Asp Leu Tyr Glu Trp Ile Glu Glu Ala
1               5                   10                  15
Arg Lys Lys Ala Ala Lys Ala
            20

<210> 23
<211> 25
<212> PRT
<213> Homo sapiens

<400> 23
Val Met Val Thr Ile Pro Ala Asp Leu Tyr Glu Trp Ile Glu Glu Gly
1               5                   10                  15
Gly Thr Trp Trp Thr Glu Trp Ser Gln
            20                  25

<210> 24
<211> 22
<212> PRT
<213> Homo sapiens

<400> 24
Val Met Val Thr Ile Pro Ala Asp Leu Tyr Glu Trp Ile Glu Glu Thr
1               5                   10                  15
Trp Trp Glu Thr Trp Trp
            20

<210> 25
<211> 20
<212> PRT
<213> Homo sapiens

<400> 25
Val Met Val Thr Ile Pro Ala Asp Leu Tyr Glu Trp Ile Glu Glu Val
1               5                   10                  15
Pro Gly Trp Gly
            20

<210> 26
<211> 20
<212> PRT
<213> Homo sapiens

<400> 26
Val Met Val Thr Ile Pro Ala Asp Leu Tyr Glu Trp Ile Glu Glu Val
1               5                   10                  15
Pro Gly Lys Gly
            20

<210> 27
<211> 20
<212> PRT
<213> Homo sapiens

<400> 27
Val Met Val Thr Ile Pro Ala Asp Leu Tyr Glu Trp Ile Glu Glu Val
1               5                   10                  15
Pro Gly Ala Gly
            20

<210> 28
<211> 31
<212> PRT
<213> Homo sapiens

<400> 28
Val Met Val Thr Ile Pro Ala Asp Leu Tyr Glu Trp Ile Glu Glu Arg
1               5                   10                  15
Gln Ile Lys Ile Trp Phe Gln Asn Arg Arg Met Lys Trp Lys Lys
            20                  25                  30

<210> 29
<211> 23
<212> PRT
<213> Homo sapiens

<400> 29

Arg Arg Arg Arg Arg Arg Arg Arg Val Met Val Thr Ile Pro Ala Asp
1               5                   10                  15
Leu Tyr Glu Trp Ile Glu Glu
            20

<210> 30
<211> 23
<212> PRT
<213> Homo sapiens


<400> 30
Val Met Val Thr Ile Pro Ala Asp Leu Tyr Glu Trp Ile Glu Glu Arg
1               5                   10                  15
Arg Arg Arg Arg Arg Arg Arg
            20

<210> 31
<211> 23
<212> PRT
<213> Homo sapiens


<400> 31
Ala Arg Lys Lys Ala Ala Lys Ala Val Met Val Thr Ile Pro Ala Asp
1               5                   10                  15
Leu Tyr Glu Trp Ile Glu Glu
            20

<210> 32
<211> 25
<212> PRT
<213> Homo sapiens


<400> 32
Gly Gly Thr Trp Trp Thr Glu Trp Ser Gln Val Met Val Thr Ile Pro
1               5                   10                  15
Ala Asp Leu Tyr Glu Trp Ile Glu Glu
            20                  25

<210> 33
<211> 25
<212> PRT
<213> Homo sapiens


<400> 33
Gly Gly Lys Glu Thr Trp Trp Glu Thr Trp Val Met Val Thr Ile Pro
1               5                   10                  15
Ala Asp Leu Tyr Glu Trp Ile Glu Glu
            20                  25

<210> 34
<211> 20
<212> PRT
<213> Homo sapiens


<400> 34
Val Pro Gly Trp Gly Val Met Val Thr Ile Pro Ala Asp Leu Tyr Glu
1               5                   10                  15
Trp Ile Glu Glu

            20

<210> 35
<211> 20
<212> PRT
<213> Homo sapiens


<400> 35
Val Pro Gly Ala Gly Val Met Val Thr Ile Pro Ala Asp Leu Tyr Glu
1               5                   10                  15
Trp Ile Glu Glu
            20

<210> 36
<211> 20
<212> PRT
<213> Homo sapiens


<400> 36
Val Pro Gly Lys Gly Val Met Val Thr Ile Pro Ala Asp Leu Tyr Glu
1               5                   10                  15
Trp Ile Glu Glu
            20

<210> 37
<211> 25
<212> PRT
<213> Homo sapiens


<400> 37
Val Met Val Thr Ile Pro Ala Asp Leu Tyr Glu Trp Ile Glu Glu Val
1               5                   10                  15
Pro Gly Ala Gly Val Pro Gly Ala Gly
            20                  25

<210> 38
<211> 25
<212> PRT
<213> Homo sapiens


<400> 38
Val Pro Gly Ala Gly Val Pro Gly Ala Gly Val Met Val Thr Ile Pro
1               5                   10                  15
Ala Asp Leu Tyr Glu Trp Ile Glu Glu
            20                  25

<210> 39
<211> 20
<212> PRT
<213> Homo sapiens


<400> 39
Val Pro Gly Asp Gly Val Met Val Thr Ile Pro Ala Asp Leu Tyr Glu
1               5                   10                  15
Trp Ile Glu Glu
            20

<210> 40

<211> 20
<212> PRT
<213> Homo sapiens

<400> 40
Val Met Val Thr Ile Pro Ala Asp Leu Tyr Glu Trp Ile Glu Glu Val
1               5                   10                  15
Pro Gly Asp Gly
            20

<210> 41
<211> 16
<212> PRT
<213> Homo sapiens

<400> 41
Asp Leu Ser Ala Val Cys Trp Ala Phe Pro Trp Asp Pro Glu Cys His
1               5                   10                  15

<210> 42
<211> 15
<212> PRT
<213> Homo sapiens

<400> 42
Ile Met Val Thr Ile Pro Ala Asp Leu Tyr Glu Trp Ile Glu Glu
1               5                   10                  15

<210> 43
<211> 16
<212> PRT
<213> Homo sapiens

<400> 43
Ile Met Val Thr Ile Pro Ala Asp Leu Tyr Glu Trp Ile Glu Glu Gln
1               5                   10                  15

<210> 44
<211> 8
<212> PRT
<213> Homo sapiens

<400> 44
Ala Arg Lys Lys Ala Ala Lys Ala
1               5

<210> 45
<211> 8
<212> PRT
<213> Homo sapiens

<400> 45
Thr Trp Trp Thr Glu Trp Ser Gln

1                   5

<210> 46
<211> 7
<212> PRT
<213> Homo sapiens


<400> 46
Thr Trp Trp Glu Thr Trp Trp
1               5

<210> 47
<211> 5
<212> PRT
<213> Homo sapiens


<400> 47
Val Pro Gly Trp Gly
1               5

<210> 48
<211> 8
<212> PRT
<213> Homo sapiens


<400> 48
Lys Glu Thr Trp Trp Glu Thr Trp
1               5

<210> 49
<211> 5
<212> PRT
<213> Homo sapiens


<400> 49
Val Pro Gly Lys Gly
1               5

<210> 50
<211> 5
<212> PRT
<213> Homo sapiens


<400> 50
Val Pro Gly Ala Gly
1               5

<210> 51
<211> 16
<212> PRT
<213> Homo sapiens


<400> 51
Arg Gln Ile Lys Ile Trp Phe Gln Asn Arg Arg Met Lys Trp Lys Lys
1               5                   10                  15

<210> 52
<211> 8
<212> PRT
<213> Homo sapiens

<400> 52
Arg Arg Arg Arg Arg Arg Arg Arg
1               5

<210> 53
<211> 5
<212> PRT
<213> Homo sapiens

<400> 53
Val Pro Gly Asp Gly
1               5

<210> 54
<211> 5
<212> PRT
<213> Homo sapiens

<400> 54
Val Pro Gly Xaa Gly
1               5

<210> 55
<211> 4
<212> PRT
<213> Homo sapiens

<400> 55
Ile Pro Gly Gly
1

<210> 56
<211> 5
<212> PRT
<213> Homo sapiens

<400> 56
Ala Val Gly Val Pro
1               5

<210> 57
<211> 5
<212> PRT
<213> Homo sapiens

<400> 57
Ile Pro Gly Xaa Gly
1               5

<210> 58
<211> 4

<212> PRT
<213> Homo sapiens


<400> 58
Pro Gly Val Gly
1

<210> 59
<211> 5
<212> PRT
<213> Homo sapiens


<400> 59
Leu Pro Gly Xaa Gly
1               5

<210> 60
<211> 5
<212> PRT
<213> Homo sapiens


<400> 60
Leu Pro Gly Val Gly
1               5

<210> 61
<211> 6
<212> PRT
<213> Homo sapiens


<400> 61
Val Ala Pro Gly Val Gly
1               5

<210> 62
<211> 5
<212> PRT
<213> Homo sapiens


<400> 62
Xaa Pro Gly Val Gly
1               5

<210> 63
<211> 8
<212> PRT
<213> Homo sapiens


<400> 63
Gly Val Gly Val Pro Gly Val Gly
1               5

<210> 64
<211> 9
<212> PRT
<213> Homo sapiens

<400> 64
Val Pro Gly Phe Gly Val Gly Ala Gly
1               5

<210> 65
<211> 8
<212> PRT
<213> Homo sapiens


<400> 65
Val Pro Gly Gly Val Pro Gly Gly
1               5

<210> 66
<211> 15
<212> PRT
<213> Homo sapiens


<400> 66
Ile Val Val Thr Met Pro Ala Asp Leu Trp Asp Trp Ile Lys Ala
1               5                   10                  15

<210> 67
<211> 15
<212> PRT
<213> Homo sapiens


<400> 67
Ile Val Val Thr Met Pro Ala Asp Leu Trp Asp Trp Ile Arg Ala
1               5                   10                  15

<210> 68
<211> 15
<212> PRT
<213> Homo sapiens


<400> 68
Ile Val Val Thr Met Pro Ala Asp Leu Trp Asp Trp Ile Arg Lys
1               5                   10                  15

<210> 69
<211> 15
<212> PRT
<213> Homo sapiens


<400> 69
Ile Val Val Thr Met Pro Ala Asp Leu Trp Asp Trp Ile Ala Ala
1               5                   10                  15

<210> 70
<211> 15
<212> PRT
<213> Homo sapiens


<400> 70
Ile Val Val Thr Met Pro Ala Asp Leu Trp Asp Trp Ala Arg Ala

1               5                   10                  15

<210> 71
<211> 15
<212> PRT
<213> Homo sapiens


<400> 71
Ile Val Val Thr Met Pro Ala Asp Leu Trp Ala Trp Ile Arg Ala
1               5                   10                  15

<210> 72
<211> 15
<212> PRT
<213> Homo sapiens


<400> 72
Ile Val Val Thr Met Pro Ala Asp Leu Ala Asp Trp Ile Arg Ala
1               5                   10                  15

<210> 73
<211> 15
<212> PRT
<213> Homo sapiens


<400> 73
Ile Val Val Thr Met Pro Ala Asp Ala Trp Asp Trp Ile Arg Ala
1               5                   10                  15

<210> 74
<211> 15
<212> PRT
<213> Homo sapiens


<400> 74
Ile Val Val Thr Met Pro Ala Asp Leu Trp Asp Trp Ile Arg Ala
1               5                   10                  15

<210> 75
<211> 15
<212> PRT
<213> Homo sapiens


<400> 75
Ile Val Val Thr Ala Pro Ala Asp Leu Trp Asp Trp Ile Arg Ala
1               5                   10                  15

<210> 76
<211> 15
<212> PRT
<213> Homo sapiens


<400> 76
Ile Val Val Ala Met Pro Ala Asp Leu Trp Asp Trp Ile Arg Ala
1               5                   10                  15

<210> 77

<211> 15
<212> PRT
<213> Homo sapiens

<400> 77
Ile Ala Val Thr Met Pro Ala Asp Leu Trp Asp Trp Ile Arg Ala
1               5                   10                  15

<210> 78
<211> 15
<212> PRT
<213> Homo sapiens

<400> 78
Ala Val Val Thr Met Pro Ala Asp Leu Trp Asp Trp Ile Arg Ala
1               5                   10                  15

<210> 79
<211> 15
<212> PRT
<213> Homo sapiens

<400> 79
Ile His Val Thr Met Pro Ala Asp Leu Trp Asp Trp Ile Arg Ala
1               5                   10                  15

<210> 80
<211> 15
<212> PRT
<213> Homo sapiens

<400> 80
Ile Gln Val Thr Met Pro Ala Asp Leu Trp Asp Trp Ile Arg Ala
1               5                   10                  15

<210> 81
<211> 15
<212> PRT
<213> Homo sapiens

<400> 81
Ile Arg Val Thr Met Pro Ala Asp Leu Trp Asp Trp Ile Arg Ala
1               5                   10                  15

<210> 82
<211> 15
<212> PRT
<213> Homo sapiens

<400> 82
Ile Thr Val Thr Met Pro Ala Asp Leu Trp Asp Trp Ile Arg Ala
1               5                   10                  15

<210> 83
<211> 15
<212> PRT
<213> Homo sapiens

<400> 83
Ile Trp Val Thr Met Pro Ala Asp Leu Trp Asp Trp Ile Arg Ala
1               5                   10                  15

<210> 84
<211> 15
<212> PRT
<213> Homo sapiens


<400> 84
Ile Tyr Val Thr Met Pro Ala Asp Leu Trp Asp Trp Ile Arg Ala
1               5                   10                  15

<210> 85
<211> 15
<212> PRT
<213> Homo sapiens


<400> 85
Ile Val Val Thr Ile Pro Ala Asp Leu Trp Asp Trp Ile Arg Ala
1               5                   10                  15

<210> 86
<211> 15
<212> PRT
<213> Homo sapiens


<400> 86
Ile Val Val Thr Leu Pro Ala Asp Leu Trp Asp Trp Ile Arg Ala
1               5                   10                  15

<210> 87
<211> 15
<212> PRT
<213> Homo sapiens


<400> 87
Ile Val Val Thr Val Pro Ala Asp Leu Trp Asp Trp Ile Arg Ala
1               5                   10                  15

<210> 88
<211> 15
<212> PRT
<213> Homo sapiens


<400> 88
Ile Val Val Thr Met Pro Ala Asp Leu Trp Asp Trp Ile Met Ala
1               5                   10                  15

<210> 89
<211> 15
<212> PRT
<213> Homo sapiens


<400> 89

Ile Val Val Thr Met Pro Ala Asp Leu Trp Asp Trp Ile Asn Ala
1               5                   10                  15

<210> 90
<211> 15
<212> PRT
<213> Homo sapiens


<400> 90
Ile Val Val Thr Met Pro Ala Asp Leu Trp Asp Trp Ile Gln Ala
1               5                   10                  15

<210> 91
<211> 15
<212> PRT
<213> Homo sapiens


<400> 91
Ile Val Val Thr Ile Pro Ala Asp Leu Trp Asp Trp Ile Arg Ala
1               5                   10                  15

<210> 92
<211> 15
<212> PRT
<213> Homo sapiens


<400> 92
Ile Val Val Thr Leu Pro Ala Asp Leu Trp Asp Trp Ile Arg Ala
1               5                   10                  15

<210> 93
<211> 15
<212> PRT
<213> Homo sapiens


<400> 93
Ile His Val Thr Ile Pro Ala Asp Leu Trp Asp Trp Ile Asn Lys
1               5                   10                  15

<210> 94
<211> 14
<212> PRT
<213> Homo sapiens


<400> 94
Ile His Val Thr Ile Pro Ala Asp Leu Trp Asp Trp Ile Asn
1               5                   10

<210> 95
<211> 13
<212> PRT
<213> Homo sapiens


<400> 95
Ile His Val Thr Ile Pro Ala Asp Leu Trp Asp Trp Ile
1               5                   10

<210> 96
<211> 20
<212> PRT
<213> Homo sapiens

<400> 96
Asp Ser Ser Ala Val Cys Trp Ala Phe Pro His His Pro Leu Cys His
1               5                   10                  15
Met Lys Ala Thr
            20

<210> 97
<211> 16
<212> PRT
<213> Homo sapiens

<400> 97
Ala Asp Ala Asp Met Cys Trp Phe Phe Pro Thr Ser Pro Trp Cys His
1               5                   10                  15

<210> 98
<211> 17
<212> PRT
<213> Homo sapiens

<400> 98
Asp Leu Ser Ala Val Cys Trp Ala Phe Pro Trp Asp Pro Glu Cys His
1               5                   10                  15
Met

<210> 99
<211> 16
<212> PRT
<213> Homo sapiens

<400> 99
Asp Ser Ser Ala Val Cys Trp Ala Phe Pro Tyr Leu Pro Glu Cys His
1               5                   10                  15

<210> 100
<211> 16
<212> PRT
<213> Homo sapiens

<400> 100
Asp Ile Ser Ala Val Cys Trp Ala Phe Pro Phe Asp Pro Glu Cys His
1               5                   10                  15

<210> 101
<211> 20
<212> PRT

<213> Homo sapiens

<400> 101
Ala Tyr Glu Cys Pro Arg Leu Glu Tyr Asp Met Phe Gly Ala Leu His
1               5                   10                  15
Cys Leu Pro Ser
            20

<210> 102
<211> 15
<212> PRT
<213> Homo sapiens

<400> 102
Cys Pro Arg Leu Glu Tyr Asp Met Phe Gly Ala Leu His Cys Leu
1               5                   10                  15

<210> 103
<211> 15
<212> PRT
<213> Homo sapiens

<400> 103
Cys Leu Asp Leu Gln Tyr Asp Pro Trp Gly Ala Leu His Cys Ile
1               5                   10                  15

<210> 104
<211> 15
<212> PRT
<213> Homo sapiens

<400> 104
Cys Phe Asp Leu Gln Tyr Asp Pro Trp Gly Ala Leu His Cys Ile
1               5                   10                  15

<210> 105
<211> 15
<212> PRT
<213> Homo sapiens

<400> 105
Cys Leu Asp Leu Gln Tyr Asp Met Phe Gly Ala Leu His Cys Val
1               5                   10                  15

<210> 106
<211> 15
<212> PRT
<213> Homo sapiens

<400> 106
Cys Leu Asp Leu Val Tyr Asp Pro Trp Gly Ala Leu His Cys Ile
1               5                   10                  15

<210> 107
<211> 15
<212> PRT
<213> Homo sapiens

<400> 107
Cys Trp Val Leu Glu Tyr Asp Met Phe Gly Ala Leu His Cys Arg
1               5                   10                  15

<210> 108
<211> 15
<212> PRT
<213> Homo sapiens


<400> 108
Cys Trp Ala Leu Glu Tyr Asp Met Phe Gly Tyr Leu His Cys Arg
1               5                   10                  15

<210> 109
<211> 15
<212> PRT
<213> Homo sapiens


<400> 109
Cys Trp Val Leu Glu Tyr Asp Met Phe Gly Phe Leu His Cys Arg
1               5                   10                  15

<210> 110
<211> 15
<212> PRT
<213> Homo sapiens


<400> 110
Cys Trp Val Leu Glu Tyr Asp Met Phe Gly Tyr Leu His Cys Arg
1               5                   10                  15

<210> 111
<211> 15
<212> PRT
<213> Homo sapiens


<400> 111
Val His Val Thr Ile Pro Ala Glu Leu Trp Glu Trp Val Arg Arg
1               5                   10                  15

<210> 112
<211> 15
<212> PRT
<213> Homo sapiens


<400> 112
Val His Phe Thr Ile Pro Ala Asp Leu Trp Glu Trp Val Arg Arg
1               5                   10                  15

<210> 113
<211> 15
<212> PRT
<213> Homo sapiens


<400> 113

Val His Val Gln Ile Pro Ala Asp Leu Trp Glu Trp Val Arg Arg
1               5                   10                  15

<210> 114
<211> 15
<212> PRT
<213> Homo sapiens


<400> 114
Val His Val Thr Phe Pro Ala Asp Leu Trp Glu Trp Val Arg Arg
1               5                   10                  15

<210> 115
<211> 15
<212> PRT
<213> Homo sapiens


<400> 115
Val His Val Thr Ile Pro Gln Asp Leu Trp Glu Trp Val Arg Arg
1               5                   10                  15

<210> 116
<211> 15
<212> PRT
<213> Homo sapiens


<400> 116
Val His Val Thr Ile Pro Ala Asn Leu Trp Glu Trp Val Arg Arg
1               5                   10                  15

<210> 117
<211> 15
<212> PRT
<213> Homo sapiens


<400> 117
Val His Val Thr Ile Pro Ala Asp Phe Trp Glu Trp Val Arg Arg
1               5                   10                  15

<210> 118
<211> 15
<212> PRT
<213> Homo sapiens


<400> 118
Val His Val Thr Ile Pro Ala Asp Leu Tyr Glu Trp Val Arg Arg
1               5                   10                  15

<210> 119
<211> 15
<212> PRT
<213> Homo sapiens


<400> 119
Val His Val Thr Ile Pro Ala Asp Leu Trp Asn Trp Val Arg Arg
1               5                   10                  15

<210> 120
<211> 15
<212> PRT
<213> Homo sapiens

<400> 120
Val His Val Thr Ile Pro Ala Asp Leu Trp Glu Phe Val Arg Arg
1               5                   10                  15

<210> 121
<211> 15
<212> PRT
<213> Homo sapiens

<400> 121
Val His Val Thr Ile Pro Ala Asp Leu Trp Glu Trp Phe Arg Arg
1               5                   10                  15

<210> 122
<211> 15
<212> PRT
<213> Homo sapiens

<400> 122
Val His Val Tyr Ile Pro Ala Glu Leu Trp Glu Trp Val Arg Arg
1               5                   10                  15

<210> 123
<211> 15
<212> PRT
<213> Homo sapiens

<400> 123
Val His Val Thr Ile Pro Ala Glu Trp Trp Glu Trp Val Arg Arg
1               5                   10                  15

<210> 124
<211> 15
<212> PRT
<213> Homo sapiens

<400> 124
Val His Phe Thr Phe Pro Gln Asp Leu Trp Glu Trp Val Arg Arg
1               5                   10                  15

<210> 125
<211> 15
<212> PRT
<213> Homo sapiens

<400> 125
Val His Phe Thr Phe Pro Gln Asp Phe Trp Glu Trp Val Arg Arg
1               5                   10                  15

<210> 126
<211> 15
<212> PRT

<213> Homo sapiens

<400> 126
Val His Phe Thr Ile Pro Gln Asp Leu Tyr Glu Trp Val Arg Arg
1               5                   10                  15

<210> 127
<211> 15
<212> PRT
<213> Homo sapiens

<400> 127
Val His Phe Thr Phe Pro Gln Asp Leu Trp Asn Trp Val Arg Arg
1               5                   10                  15

<210> 128
<211> 15
<212> PRT
<213> Homo sapiens

<400> 128
Val His Phe Thr Phe Pro Gln Asp Leu Trp Glu Phe Val Arg Arg
1               5                   10                  15

<210> 129
<211> 15
<212> PRT
<213> Homo sapiens

<400> 129
Val His Phe Thr Phe Pro Gln Asp Leu Trp Glu Trp Phe Arg Arg
1               5                   10                  15

<210> 130
<211> 15
<212> PRT
<213> Homo sapiens

<400> 130
Val His Phe Gln Phe Pro Ala Asp Leu Trp Glu Trp Val Arg Arg
1               5                   10                  15

<210> 131
<211> 15
<212> PRT
<213> Homo sapiens

<400> 131
Val His Phe Gln Phe Pro Ala Asp Phe Trp Glu Trp Val Arg Arg
1               5                   10                  15

<210> 132
<211> 15
<212> PRT
<213> Homo sapiens

<400> 132
Val His Phe Gln Phe Pro Ala Asp Leu Tyr Glu Trp Val Arg Arg
1               5                   10                  15

<210> 133
<211> 15
<212> PRT
<213> Homo sapiens


<400> 133
Val His Phe Gln Phe Pro Ala Asp Leu Trp Asn Trp Val Arg Arg
1               5                   10                  15

<210> 134
<211> 15
<212> PRT
<213> Homo sapiens


<400> 134
Val His Phe Gln Phe Pro Ala Asp Leu Trp Glu Phe Val Arg Arg
1               5                   10                  15

<210> 135
<211> 15
<212> PRT
<213> Homo sapiens


<400> 135
Val His Phe Gln Phe Pro Ala Asp Leu Trp Glu Trp Phe Arg Arg
1               5                   10                  15

<210> 136
<211> 15
<212> PRT
<213> Homo sapiens


<400> 136
Val His Phe Gln Phe Pro Gln Asp Trp Trp Glu Trp Val Arg Arg
1               5                   10                  15

<210> 137
<211> 15
<212> PRT
<213> Homo sapiens


<400> 137
Val His Phe Gln Ile Pro Gln Asp Trp Trp Glu Trp Val Arg Arg
1               5                   10                  15

<210> 138
<211> 15
<212> PRT
<213> Homo sapiens


<400> 138
Val His Phe Gln Phe Pro Gln Asp Trp Tyr Glu Trp Val Arg Arg
1               5                   10                  15

<210> 139
<211> 15
<212> PRT
<213> Homo sapiens

<400> 139
Val His Phe Gln Phe Pro Gln Asp Trp Trp Asn Trp Val Arg Arg
1               5                   10                  15

<210> 140
<211> 15
<212> PRT
<213> Homo sapiens

<400> 140
Val His Phe Gln Phe Pro Gln Asp Leu Trp Glu Phe Val Arg Arg
1               5                   10                  15

<210> 141
<211> 15
<212> PRT
<213> Homo sapiens

<400> 141
Val His Phe Gln Phe Pro Gln Asp Trp Trp Glu Trp Phe Arg Arg
1               5                   10                  15

<210> 142
<211> 15
<212> PRT
<213> Homo sapiens

<400> 142
Val His Phe Thr Ile Pro Ala Asp Phe Trp Glu Trp Phe Arg Arg
1               5                   10                  15

<210> 143
<211> 15
<212> PRT
<213> Homo sapiens

<400> 143
Val His Val Gln Ile Pro Ala Asp Phe Trp Glu Trp Phe Arg Arg
1               5                   10                  15

<210> 144
<211> 15
<212> PRT
<213> Homo sapiens

<400> 144
Val His Val Thr Phe Pro Ala Asp Leu Trp Glu Trp Phe Arg Arg
1               5                   10                  15

<210> 145
<211> 15

<212> PRT
<213> Homo sapiens

<400> 145
Val His Val Thr Ile Pro Gln Asp Phe Trp Glu Trp Phe Arg Arg
1               5                   10                  15

<210> 146
<211> 15
<212> PRT
<213> Homo sapiens

<400> 146
Val His Phe Thr Ile Pro Gln Asp Trp Trp Glu Trp Val Arg Arg
1               5                   10                  15

<210> 147
<211> 15
<212> PRT
<213> Homo sapiens

<400> 147
Val His Phe Thr Phe Pro Gln Asp Leu Tyr Asn Trp Val Arg Arg
1               5                   10                  15

<210> 148
<211> 15
<212> PRT
<213> Homo sapiens

<400> 148
Val His Phe Thr Phe Pro Gln Asp Leu Tyr Asn Phe Val Arg Arg
1               5                   10                  15

<210> 149
<211> 15
<212> PRT
<213> Homo sapiens

<400> 149
Val His Val Thr Ile Pro Ala Asp Leu Tyr Asn Phe Phe Arg Arg
1               5                   10                  15

<210> 150
<211> 15
<212> PRT
<213> Homo sapiens

<400> 150
Val His Phe Gln Phe Pro Gln Asp Leu Trp Glu Trp Val Arg Arg
1               5                   10                  15

<210> 151
<211> 14
<212> PRT
<213> Homo sapiens

<400> 151
Val His Phe Thr Ile Pro Gln Asp Leu Tyr Asn Trp Arg Arg
1               5                   10

<210> 152
<211> 15
<212> PRT
<213> Homo sapiens

<400> 152
Val His Phe Thr Ile Pro Ala Asp Leu Tyr Asn Phe Val Arg Arg
1               5                   10                  15

<210> 153
<211> 15
<212> PRT
<213> Homo sapiens

<400> 153
Val His Phe Gln Ile Pro Gln Asp Leu Tyr Asn Phe Phe Arg Arg
1               5                   10                  15

<210> 154
<211> 15
<212> PRT
<213> Homo sapiens

<400> 154
Val His Phe Gln Phe Pro Gln Glu Trp Tyr Asn Trp Phe Arg Arg
1               5                   10                  15

<210> 155
<211> 15
<212> PRT
<213> Homo sapiens

<400> 155
Val Arg Phe Gln Phe Gly Gln Glu Trp Tyr Asn Phe Phe Arg Arg
1               5                   10                  15

<210> 156
<211> 15
<212> PRT
<213> Homo sapiens

<400> 156
Val Pro Gly Ala Gly Val Pro Gly Ala Gly Ile His Val Thr Ile
1               5                   10                  15

<210> 157
<211> 17
<212> PRT
<213> Homo sapiens

<400> 157
Val Pro Gly Ala Gly Val Pro Gly Ala Gly Ile His Val Thr Ile Pro

1               5                   10                  15
Ala

## Description

The invention relates to IL-17A binding polypeptides, inhibitor of the complex IL17A/IL17RA/IL17RC interaction, and the corresponding bioconjugates, their pharmaceutical composition and the medical use thereof in the treatment of inflammatory and autoimmune ophthalmic diseases.

### Background of the invention

Human cytotoxic T lymphocyte-associated antigen 8 (CTLA8), named also IL-17 was identified in 1993. The first bioactivity of human IL-17 showed the production of IL-6 and IL-8 from rheumatoid arthritis synoviocytes in response to IL-17. This suggested a role of IL-17 in inflammation through IL-6 and in neutrophil recruitment through IL-8. The interleukin 17 (IL-17) family, a subset of cytokines consisting of six members, namely IL-17A-F, plays crucial roles in host defense against microbial organisms and in the development of inflammatory diseases. IL-17A and F are the closest members, with 56% homology. They are secreted as IL-17A and IL-17F homodimers and as IL-17A/F heterodimers. Sharing the greatest sequence homology (56%) with IL-17A, IL-17F also signals through the same receptor complex, although IL-17F binds to IL-17RA with ∼100 to 1000 times lower affinity than does IL-17A, while the binding affinities for IL-17RC is comparable between the two cytokines (Onishi and Gaffen 2010, Gu, Wu et al. 2013). IL-17B, IL-17C, and IL-17D are defined as pro-inflammatory cytokines but their role is not fully known. IL-17E, also known as IL-25, has the lowest homology and is involved in Th2 cell responses against parasites and allergy. CCL20 drives the recruitment of Th17 and dendritic cells to the inflammatory site. In turn, Th17 cells are activated and produce inflammatory mediators leading to chronic inflammation.

As regarding IL17 family receptors, IL-17 receptor family now consists of 5 members (IL-17RA, RB, RC, RD and RE), all of which, like their ligands, share sequence homology. IL-17RA is ubiquitously expressed on a wide range of tissues and cell types. Upon the stimulation with IL-17, IL-17RA initiates the activation of downstream signaling pathways to induce the production of pro-inflammatory molecules. However, IL-17RA alone is insufficient to mediated IL-17signaling.

Further evaluation revealed that IL-17 signals through a heterodimeric receptor complex composed of IL-17RA and IL-17RC It is proposed that the binding of ligand to the first IL-17RA receptor subunit alters the affinity and specificity of the second binding event, thereby promoting the formation of a heterodimeric rather than a homodimeric receptor complex. The most widely investigated cytokine of this family, IL-17A, is a pro-inflammatory cytokine that plays an essential role in host defense against microbial infections and is implicated in various inflammatory conditions such as autoimmune diseases, metabolic disorders, and cancer. Pathological production of IL-17A leads to excessive inflammation and overt tissue damage.

Through the production of a variety of molecules including cytokines, chemokines, acute phase proteins, anti-microbial peptides, mucins, and matrix metalloproteinases, IL-17A can propagate cascades of events that lead to neutrophil recruitment, inflammation and host defense. Although IL-17A is the signature cytokine produced by T helper 17 (Th17) cells, IL-17A and other IL-17 family cytokines have multiple sources ranging from immune cells to nonimmune cells. The IL-17 family signals via their correspondent receptors and activates downstream pathways that include NFκB, MAPKs and C/EBPs to induce the expression of antimicrobial peptides, cytokines and chemokines. The proximal adaptor Act1 is a common mediator during the signaling of all IL-17 cytokines so far and is thus involved in IL-17 mediated host defense and IL-17-driven autoimmune conditions.

Inflammatory and autoimmune eye disease is the commonest cause of acquired visual disability in the working age population, such as infectious corneal ulcers, endophthalmitis, autoimmune diseases of the cornea, ocular surface, keratoconjunctivitis sicca, vernal keratoconjunctivitis, stromal herpetic keratitis, corneal allograft rejection, corneal infections and dry eye.

The current treatment of said eye diseases is made by the use of non-specific anti-inflammatory agents, such as corticosteroids or anti-oxidants.

Among inflammatory and autoimmune diseases involving Th-17 cells and IL17 pathway activation, dry eye is one of the commonest cause of acquired visual impairment in adult population (Stern, Schaumburg et al. 2010, Stevenson, Chauhan et al. 2012).

Dry eye disease (DED) is one of the most common ophthalmic pathologies, it is a multifactorial autoimmune disorder of the tear system and ocular surface with a poorly known pathogenesis that result in discomfort, visual disturbance and tear film impairment. Current treatment of dry eye is mainly symptomatic, in fact it is treated by ocular lubrificants and non-specific anti-inflammatory agents as corticosteroids, cyclosporine A, tacrolimus. Since reactive oxygen species are also involved in ocular cell injury and death, also antioxidants as ascorbic acid can be useful in the treatment of DED (Parul Singh 2012, Ma, Siegfried et al. 2016, Hah, Chung et al. 2017). The disease is characterized by an immune and inflammatory process that affects the ocular surface and, in severe cases, may lead to blindness due to corneal ulceration or scarring. The ocular surface inflammation in DED is sustained by ongoing activation and infiltration of pathogenic immune cells, primarily of the CD4T cell compartment. T cells involvement in the immunopathogenesis of DED is supported by many observations as increased T cell infiltration of the conjunctiva in both clinical and experimental dry eye or the presence of IFN-T cells in the conjunctiva of dry eye mice; additionally, DED can be induced in mice by transfer of CD4T cells from dry eye affected mice. Topical application of T cell immunosuppressants, such as cyclosporine lead to disease remission.

A functional balance between Tregs and effector T cells is important for maintaining efficient immune responses while avoiding autoimmunity. CD4CD25Tregs are a naturally occurring suppressor T cell population that inhibits the activity of self-reactive Th1, Th2, and Th17 cells that potentially induce inflammation and tissue damaging. An imbalanced presence of Tregs, particularly Th17 cells, has been demonstrated in DED, while in vivo blockade of IL-17 can ameliorate the disease and prevent both Th17 cell differentiation and loss of Treg function (Chauhan, El Annan et al. 2009, Chen, Chauhan et al. 2014).

It has been observed that Interleukin-17 (IL-17) is associated with damage to the corneal epithelial barrier function, which is the most sight threatening complication of dry eye disease. An increased expression of Th17 cell is reported in dry eye patients ocular tissues inducing an increase in IL-6, TGF-β, IL-23 and IL-17A concentration on the ocular surface of dry eye patients as well as increased concentration of IL-17 in tears and number of Th17 cells on the ocular surface of experimental dry eye models.

In addition, IL-17 is demonstrated to induce secretion of MMP-3 and -9 by epithelial cells causing a significant increase in corneal epithelial permeability which is ameliorated by in-vivo neutralization of IL-17. Th17 cells role has been depicted in dry eye pathogenesis but also the mechanism of induction of autoimmunity in the draining lymph nodes using a mouse model of dry eye, in fact, increased frequency of Th17 cells was reported in the draining lymphnode where these cells exhibit specific resistance to regulatory-T cell mediated suppression.

The functional relevance of Th17 cells in the pathogenesis of dry eye disease was fundamentally confirmed by demonstrating that in-vivo neutralization of IL-17 results in a markedly attenuated induction and severity of disease. Interestingly, dry eye amelioration was paralleled by a reduction in the expansion of Th17 cells and prevention of the loss of Treg function in the draining lymphnode (Chauhan, El Annan et al. 2009, De Paiva, Chotikavanich et al. 2009, de Paiva, Huang et al. 2014, Subbarayal, Chauhan et al. 2016). None of the currently developed IL17-A modulators has been used till now in clinics for the topical treatment of ocular pathologies as dry eye disease. Currently dry eye disease is treated mainly with calcineurine inhibitors as tacrolimus and cyclosporine A or ICAM1 and LFA1 inhibitors as lifitegrast. Calcineurin inhibitors downregulate the transcription of interleukin-2, the downstream effect of this inhibition is a reduction in T cell activation. They are cheap and currently are the first treatment line for dry eye disease. However, they have low patients' compliance (due to burning, redness, itching) and a high discontinuation rate. Their intraocular penetration is poor, and they have low acqueous solubility. Their efficacy is seen after months (because of its effect on quite naive T cells) and desensibilization and rebound effects after discontinuation are reported by patients. At the very end only 15% of patients find it useful. Lifitegrast has a different mechanism of action since it inhibits ICAM1/LFA1 interaction.

At the inflammatory sites, LFA-1 and ICAM-1 interaction leads to T-cell adhesion to endothelial cells, migration to inflamed tissue, antigen presentation, and recognition facilitating the formation of an immunological synapse. These immunological synapses facilitate the propagation of downstream signals which lead to release of inflammatory mediators, cytokines, chemokines, TNF-α, IL-1, which further intensify and perpetuate inflammation in ocular tissues. Lifitegrast is more effective than calcineurine inhibitors but it is really pricey and desensibilization effects are reported by patients with an additional bitter, long lasting taste generation after use.

As an augmented expression of IL-17A is linked with various autoimmune disorders as dry eye disease (DED), blocking of IL-17A has been of great therapeutic interest. Targeting the cytokine or its receptor with monoclonal antibodies (mAbs) is the most direct and specific strategy. The first two anti-IL-17A antibodies clinically tested with indications different from dry eye disease were secukinumab (AIN457, ConsentyxTM), a fully human IgG1k anti-IL-17A mAb, and ixekizumab (LY2439821), a humanized IgG4 antibody. Secukinumab and Ixekizumab, are currently approved for treatment of moderate to severe psoriasis. During clinical trials, both antibodies reduced the severity of the disease by at least 75% (PASI75) in 80% of the patient population. Moreover, current advanced clinical trials have shown promising results for treatment of ankylosing spondylitis and psoriatic arthritis. Other antibodies, such as CNTO 6785, CJM112, and BCD085, are now in clinical trials. Biomolecules targeting common motifs shared by IL-17A and IL-17F are also now in clinical development, including the nanobody ALX-0761 and the mAb bimekizumab. To target the IL-17 receptor, brodalumab (AMG 827) was developed, it is a fully human IgG2 that selectively blocks signaling through the IL-17RA chain of the IL-17 receptor. This antibody inhibits human IL-17A, IL-17F, and IL-17C but also IL-25. (Beringer, Noack et al. 2016).

Although oral drugs are preferred as therapeutic option for patients; inhibition of cytokines and their receptors has thus far remained the domain of antibodies use. The reason for the preference for antibodies is that cytokines, as IL-17A, constitute protein-protein interaction (PPI) targets that have been deemed to be largely undruggable as recently as a few decades ago.

In fact, PPIs interfaces are generally flat and lack deep subpockets and grooves that are usually necessary for binding of traditional organic small molecules. The interaction of IL- 17A with its receptor IL-17RA spans across a large surface area (approximately 2,200 Å2) on IL-17A. The nature of this interaction involves also discontinuous and tertiary structural epitopes that includes both the ligand and its receptor, making the discovery of small molecule inhibitors particularly difficult. Despite these challenges, in 2013, Ensemble Therapeutics announced discovery of small molecule IL-17A antagonists even if it is not known whether Ensemble compounds have entered clinical trials (Espada, Broughton et al. 2016, Ting, Tung et al. 2018).

Despite the interface between IL-17A and its receptor is broad, flat, lacking grooves that are suitable for binding traditional small molecules, it contains single, isolated residues that contribute to most of binding free energy in the interface. These "hot spots" usually cover less than 10% of the entire interface and tends to cluster in hot regions. These hot regions are known to be druggable and dynamic to facilitate binding of the protein to a set of peptides and small molecules. Two such hot regions were previously identified for IL-17A through close inspection of its structure in complex with IL-17RA. These two regions, called α-helix and β-hairpin pockets are named based on the structural determinants of IL-17RA that interact with the cytokine.

The α-helix (residues 60±62, 99, 101, 110) and β-hairpin (residues 93±95) pockets reside within the previously defined regions I and II, respectively. Both pockets are considered viable options for binding of small molecules and macrocycles. (Liu, Dakin et al. 2016, Ting, Tung et al. 2018). Eli Lilly described in 2018 the identification of two families of peptides: 18-902 and 585- 870 which are able to bind in the α-helix and β-hairpin pockets, respectively. Ensemble macrocycles and Pfizer's small molecule compounds bind to the β-hairpin pocket, suggesting that this pocket is highly druggable. Binding of Ensemble and Pfizer macrocyclic ligand antagonists to the induced pocket at the center of the IL-17A dimer have been shown to cause conformational changes that also prevent IL-17RA binding. Additionally, Pfizer discovered a high affinity IL-17A peptide antagonist (HAP). HAP, also shows activity in inhibiting the production of multiple inflammatory cytokines by primary human keratinocytes stimulated by IL-17A and TNF-α, a psoriasis translational cell model (Liu, Desharnais et al. 2016).

Recent papers shows that the N-terminal portion of the IL-17A is suitable to design binding peptides that inhibits the interaction between IL-17A and IL-17RA (Liu, Desharnais et al. 2016, Ting, Tung et al. 2018).

However, despite the publication of a high number of possible binding peptides, none of them has been tested in clinical trials till today.

There is therefore the need of obtaining more functional and specific polypeptides or bioconjugates thereof that are able to bind the N-terminal portion of IL-17 preventing the IL17RA receptor dimerization and inhibiting IL17 pathway activation thus being promising agents in the treatment of ocular diseases.

### Brief description of the figures

Figure 1. Figure 1: Dose response curves of IL17RA-IL17RC dimerization inhibition after treatment with anti-IL17A peptides. Percent of inhibition of IL17RA/IL17RC dimerization (induced by IL17A) due to treatment with different peptides, as example, is reported.
Figure 2. Shows the activity of IL-17A binding peptides on cytokine secretion. Cytokine secretion in human differentiated and activated TH17 cells with or without IL17A binding peptides treatment. IL6, IL17 and IL23 decrease in response to our active peptides treatment. Peptide P260318-01-19 activity is reported as example.
Figure 3. Metalloproteinase 3 secretion in human differentiated and activated TH17 cells with or without IL17A binding peptides treatment. P260318-01-19 is reported as an example.
Figure 4. Shows human TH17 cell viability after 24h treatment with peptides anti IL-17A in human differentiated TH17 cells. Peptide P260318-01-19 effects are reported as example.

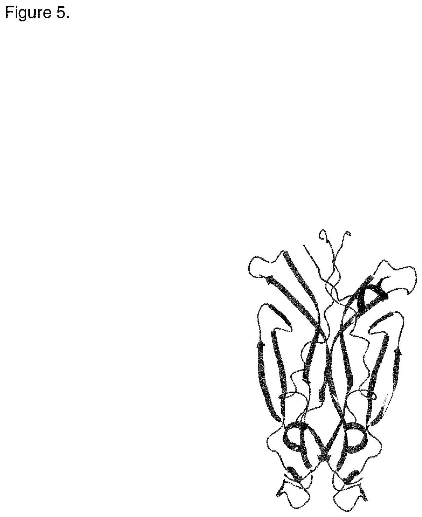

Figure 5. Shows the binding site of the IL-17A peptides.

### Definitions

Unless otherwise defined, all terms of art, notations and other scientific terminology used herein are intended to have the meanings commonly understood by those persons skilled in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "pharmaceutically acceptable excipient" refer to substances other than the active pharmaceutical ingredient (API) that have been appropriately evaluated for safety and are intentionally included in a drug delivery system. Pharmaceutically acceptable excipients are well known in the prior art and are disclosed, for example in the Handbook of Pharmaceutical Excipients, seventh edition 2013, which is included here for reference(Rowe, Sheskey et al. 2012).

Excipients are normally classified according to the function that they have in the final pharmaceutical composition. Preferably, suitable excipients according to the present invention are for example diluent, adsorbent, glidant, binder, lubricant, surfactant, disintegrating, preservatives, antioxidant or mixtures thereof.

The terms "approximately" and "about" herein refers to the range of the experimental error, which may occur in a measurement.

The terms "comprising", "having", "including" and "containing" are to be construed openended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus be included).

The terms "consists of", "consisting of" are to be construed as closed terms.

The term "biomolecule" herein refers to macromolecules that include carbohydrates, lipids, proteins or natural products.

For the purpose of the present invention "biomolecules" are selected from ascorbic acid, capronic acid, N-Acetyl-Glucosamine, N-Acetylmuramic acid, hyaluronic acid, alginic acid, chitin, (GalNAc)₂, Gal-alpha1,3-GalNAc or trigalacturonic acid.

The definition "conservative substitution" herein refers to a conservative replacement (also called a conservative mutation or a conservative substitution) is an amino acid replacement that changes a given amino acid to a different amino acid with similar biochemical properties (Simon French 1983).

### Description of the invention

The present invention regards polypeptides and their bioconjugates that are able to bind IL-17A and to block the IL-17 pathway by inhibiting the binding of IL-17A to its receptors. Said polypeptides are suitable to design innovative and specific topical treatment of ocular diseases, as dry eye, by the inhibition of IL-17A.

The invention therefore aims to the development of a topical treatment of ocular diseases as dry eye by the inhibition of IL-17A based on the use of small peptides.

In particular, it has been surprisingly found that said polypeptides are able to bind in a very precise and specific way the N-terminal portion of IL-17 that is crucial for the binding of IL-17A to its receptor.

The inhibition of said interaction is able to block the IL-17 pathway and therefore to reduce TH17 related and sustained inflammation and the subsequent damage observed in the ocular tissues, that are responsible of the of inflammatory and autoimmune ophthalmic diseases.

An object of the present invention is therefore an IL-17A binding polypeptide, inhibitor of the complex IL17A/IL17RA/IL17RC interaction, having either formula:
(I) X₁-X₂-X₃-X₄-X₅-X₆-X₇-X₈-X₉-X₁₀-X₁₁-X₁₂-X₁₃-X₁₄-X₁₅
   wherein, independently from each other:
   X₁ = I, V, D, K or W;
   X₂= H, M, K or N;
   X₃ = V or F;
   X₄ = T, Q, H or Y;
   X₅= I or F;
   X₆ = P or G;
   X₇ = A or Q;
   X₈ = D, E or N;
   X₉ = L, V, F or W;
   X₁₀ = W, Y, F or absent;
   X₁₁= D, E, N or absent;
   X₁₂ = W, F, V or absent;
   X₁₃ = I, V, F or absent;
   X₁₄ = N, R, E, F or absent;
   X₁₅ = K, R, E, F, V, W or absent;
   and with the proviso that the formula is not IHVTIPVDLWDWINK;
   or formula:
(II) aₙ-DLSAVCWAFPWDPECH-b_{n'}
   wherein, independently from each other:
   a, b = alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine or valine;
   n = 0 to 3;
   n' = 0 to 3;
   and each of the amino acid different from a or b can be replaced with a conservative substitution.

Preferably in said IL-17A binding polypeptide of formula (I):
X₁ = I or V;
X₂= H or M;
X₃ = V;
X₄ = T;
X₅ = I;
X₆ = P;
X₇ = A;
X₈ = D;
X₉ = L;
X₁₀ = W or Y;
X₁₁= D or E;
X₁₂ = W;
X₁₃ = I or V;
X₁₄ = N, R or E;
X₁₅ = K, R or E;

According to a preferred embodiment, the IL-17A binding polypeptide of formula (I) or (II) are present in form of dimers, wherein said polypeptide are linked with polyethylene glycol (PEG) at the N-terminal or at the amino acid X₄, X₇ or X₁₄ of the peptide of formula (I).

Preferably, said IL-17A binding polypeptide have an amino acid sequence that have at least 80% of identity to the sequence of formula (I) or (II), more preferably at least 85-90% of identity, even more preferably al least 93%, 95%, 96%, 97%, 98% or 99% of identity.

The present invention further relates to a bioconjugate comprising the IL-17A binding polypeptide sequence according to anyone of formula I or II above depicted, having the following formula:
(III) [Biomolecule or CAP]ₐ - [Linker]_{b} - (Sequence A)ₙ-Aₘ-[Peptide] - A'_{m'}-(Sequence A)_{n'} -[Linker]_{b'} - [Biomolecule or CAP]_{a'}
   wherein, independently from each other:
   a = 0 or 1;
   b = 0 or 1 ;
   a' = 0 or 1;
   b' = 0 or 1;
   m = 0 to 10;
   m'= 0 to 10;
   n= 0 or 5;
   n'= 0 or 5,
   and
   Peptide is the polypeptide according to formula (I) or formula (II);
   A or A' = one amino acid selected from Arg, Lys, Gly, Glu or Ala repeated m or m' times;
   and the Sequence A comprises the sequence of formula:
(IV) -Y₁-Y₂-Y₃-Y₄-Y₅-Y₆-Y₇-Y₈-Y₉-Y₁₀-Y₁₁-Y₁₂₋Y₁₃-Y₁₄-Y₁₅-Y₁₆-
   wherein:
   Y₁= A, T, V, K, R, I, L, X or G;
   Y₂= R, W, P, E, Q or A;
   Y₃= K, W, G, T, I, R or P;
   Y₄= K, T, E, W, A, R, D, G, X or F;
   Y₅= A, E, T, G, W, I, R, P or V;
   Y₆= A, W, E, R, G, P or absent;
   Y₇=K, S, W, T, F, R, V, G or absent;
   Y₈= A, Q, W, R, G or absent;
   Y₉ = N, G or absent;
   Y₁₀ = R or absent;
   Y₁₁ = R or absent;
   Y₁₂ = M or absent;
   Y₁₃ = K or absent;
   Y₁₄ = W or absent;
   Y₁₅ = K or absent;
   Y₁₆ = K or absent.
   and
   Biomolecule is, independently from each other, capronic acid, ascorbic acid, NAG-NAM, NAG, NAM, hyaluronic acid, alginic acid, chitin, (GalNAc)₂, Gal-alpha1,3-GalNAc or trigalacturonic acid;
   Cap is, independently from each other, amide, aldehyde, ester, p-Nitroanilide, 7-Amino-4-Methylcoumarin, acetyl, formyl, pyroglutamyl or a fatty acid;
   Linker is, independently from each other, 4-aminobutyric acid, beta-alaninine, 2-aminoethoxy-acetic acid, 5-aminovaleric acid, 6-aminocaproic acid, 8-Amino-3,6-dioxaoctanoic acid, 12-amino-4,7,10-trioxadodecanoic acid, 15-amino-4,7,10,13-tetraoxapenta-decanoic acid or trioxatridecan-succinamic acid.

According to a preferred embodiment, said Sequence A is attached to the N-terminal or to the C-terminal of the polypeptide of formula (I) or (II).

The term "bioconjugate" herein refers to a molecule wherein a stable covalent link between two molecules is present. Preferably, in said bioconjugate at least one biomolecule is present.

Some examples of the possible bioconjugates according to the present invention are reported here below, but all the other possible combination comprised in the formula (III) can be present:
- [Biomolecule or Cap] - [Linker] - (Sequence A) -A-[Peptide]-;
- [Biomolecule or Cap] - (Sequence A) -A-[Peptide]-;
- [Biomolecule or Cap] - -A-[Peptide]-;
- [Biomolecule or Cap] -[Peptide]-;
- -[Peptide] - A_{m'}- (Sequence A)_{n'}- [Biomolecule or Cap]_{a';}
- -[Peptide] - A_{m'}- [Biomolecule or Cap ]_{a';}
- -[Peptide]- [Biomolecule or Cap]_{a';}

According to a preferred embodiment the biomolecule group of formula (III) of the present invention is selected from ascorbic acid, capronic acid, NAG or NAM.

According to a further preferred embodiment the Cap according to the present invention is a C-ter modification selected from amides, preferably N-alkyl amides, aldehydes, esters, preferably methyl and ethyl esters, p-Nitroanilide, 7-Amino-4-Methylcoumarin or N-ter modification selected from acetyl, formyl, pyroglutamyl, fatty acids, preferably capronic acid, urea, carbamate, sulfonamide or alkylamine, preferably said Cap is selected from amides, fatty acids as capronic acid and acetyl.

According to the present invention, said linker, biomolecule or Cap groups in formula (III) can be the same or different each other.

In a further preferred embodiment of the present invention the Linker of formula (III) is selected from 4-aminobutyric acid, beta-alaninine, 2-aminoethoxy-acetic acid, 5-aminovaleric acid or trioxatridecan-succinamic acid.

Preferably, when the linker is attached on one amino acid Lysine, said linker is selected from NHS-ester, isocyanates, benzoyl fluorides or carbamates.

The preferred IL-17A binding polypeptides according to the present invention are those listed in Table 1.

**Table 1. List of the preferred IL-17A binding polypeptides.**

| **SEQ ID N.** | **Peptide name** | **Sequence** |
|---|---|---|
| SEQ ID N. 1 | P260318-01-01 | IHVTIPADLWDWINK |
| SEQ ID N. 2 | P260318-01-02 | VHVTIPADLWDWINK |
| SEQ ID N. 3 | P260318-01-03 | IMVTIPADLWDWINK |
| SEQ ID N. 4 | P260318-01-04 | VMVTIPADLWDWINK |
| SEQ ID N. 5 | P260318-01-05 | IHVTIPAELWDWINK |
| SEQ ID N. 6 | P260318-01-07 | IHVTIPADLYDWINK |
| SEQ ID N. 7 | P260318-01-08 | IHVTIPADLYEWINK |
| SEQ ID N. 8 | P260318-01-09 | IHVTIPADLWDWVNK |
| SEQ ID N. 9 | P260318-01-10 | IHVTIPADLWDWIRK |
| SEQ ID N. 10 | P260318-01-11 | IHVTIPADLWDWINR |
| SEQ ID N. 11 | P260318-01-12 | IHVTIPADLWDWIEK |
| SEQ ID N. 12 | P260318-01-13 | IHVTIPADLWDWINE |
| SEQ ID N. 13 | P260318-01-14 | IHVTIPADLYEWINK |
| SEQ ID N. 14 | P260318-01-15 | IHVTIPADLWDWVRR |
| SEQ ID N. 15 | P260318-01-16 | IHVTIPADLWDWVEE |
| SEQ ID N. 16 | P260318-01-17 | VMVTIPADLYEWINK |
| SEQ ID N. 17 | P260318-01-18 | VMVTIPADLYEWIRR |
| SEQ ID N. 18 | P260318-01-19 | VMVTIPADLYEWIEE |
| SEQ ID N. 19 | P240418-01-02 | VMVTIPADLYEWIEERRRRRR |
| SEQ ID N. 20 | P240418-01-03 | VMVTIPADL YEWIEERRR |
| SEQ ID N. 21 | P240418-01-04 | VMVTIPADLYEWIEEARKKAAKA |
| SEQ ID N. 22 | P240418-01-05 | VMVTIPADLYEWIEEGGTWWTEWSQ |
| SEQ ID N.23 | P240418-01-06 | VMVTIPADLYEWIEETWWETWW |
| SEQ ID N. 24 | P240418-01-07 | VMVTIPADLYEWIEEVPGWG |
| SEQ ID N. 25 | P140518-01-01 | VMVTIPADLYEWIEEGGKETWWETW |
| SEQ ID N. 26 | P140518-01-02 | VMVTIPADLYEWIEEVPGKG |
| SEQ ID N. 27 | P140518-01-03 | VMVTIPADLYEWIEEVPGAG |
| SEQ ID N. 28 | P140518-01-05 | VMVTIPADLYEWIEERQIKIWFQNRRMKWKK |
| SEQ ID N. 29 | P140518-01-06 | RRRRRRRRVMVTIPADLYEWIEE |
| SEQ ID N. 30 | P140518-01-07 | VMVTIPADLYEWIEERRRRRRRR |
| SEQ ID N. 31 | P140518-01-08 | ARKKAAKAVMVTIPADLYEWIEE |
| SEQ ID N. 32 | P140518-01-09 | GGTWWTEWSQVMVTIPADLYEWIEE |
| SEQ ID N. 33 | P140518-01-10 | GGKETWWETWVMVTIPADLYEWIEE |
| SEQ ID N. 34 | P140518-01-11 | VPGWGVMVTIPADL YEWIEE |
| SEQ ID N. 35 | P140518-01-12 | VPGAGVMVTIPADLYEWIEE |
| SEQ ID N. 36 | P140518-01-13 | VPGKGVMVTIPADLYEWIEE |
| SEQ ID N. 37 | P140518-01-14 | VMVTIPADLYEWIEEVPGAGVPGAG |
| SEQ ID N. 38 | P140518-01-15 | VPGAGVPGAGVMVTIPADLYEWIEE |
| SEQ ID N. 39 | P140518-01-16 | VPGDGVMVTIPADLYEWIEE |
| SEQ ID N. 40 | P140518-01-17 | VMVTIPADLYEWIEEVPGDG |
| SEQ ID N. 41 | P240418-01-01 | DLSAVCWAFPWDPECH |
| SEQ ID N. 42 | Peptide 1 | IMVTIPADLYEWIEE |
| SEQ ID N. 43 | Peptide 2 | IMVTIPADLYEWIEEQ |
| SEQ ID N. 111 | P280618-02-01 | VHVTIPAELWEWVRR |
| SEQ ID N. 112 | P280618-02-02 | VHFTIPADLWEWVRR |
| SEQ ID N. 113 | P280618-02-03 | VHVQIPADLWEWVRR |
| SEQ ID N. 114 | P280618-02-04 | VHVTFPADLWEWVRR |
| SEQ ID N. 115 | P280618-02-05 | VHVTIPQDLWEWVRR |
| SEQ ID N. 116 | P280618-02-06 | VHVTIPANLWEWVRR |
| SEQ ID N. 117 | P280618-02-07 | VHVTIPADFWEWVRR |
| SEQ ID N. 118 | P280618-02-08 | VHVTIPADLYEWVRR |
| SEQ ID N. 119 | P280618-02-09 | VHVTIPADLWNWVRR |
| SEQ ID N. 120 | P280618-02-010 | VHVTIPADLWEFVRR |
| SEQ ID N. 121 | P280618-02-011 | VHVTIPADLWEWFRR |
| SEQ ID N. 122 | P280618-02-012 | VHVYIPAELWEWVRR |
| SEQ ID N. 123 | P280618-02-013 | VHVTIPAEWWEWVRR |
| SEQ ID N. 124 | P280618-02-014 | VHFTFPQDLWEWVRR |
| SEQ ID N. 125 | P280618-02-015 | VHFTFPQDFWEWVRR |
| SEQ ID N. 126 | P280618-02-016 | VHFTIPQDLYEWVRR |
| SEQ ID N. 127 | P280618-02-017 | VHFTFPQDLWNWVRR |
| SEQ ID N. 128 | P280618-02-018 | VHFTFPQDLWEFVRR |
| SEQ ID N. 129 | P280618-02-019 | VHFTFPQDLWEWFRR |
| SEQ ID N. 130 | P280618-02-020 | VHFQFPADLWEWVRR |
| SEQ ID N. 131 | P280618-02-021 | VHFQFPADFWEWVRR |
| SEQ ID N. 132 | P280618-02-022 | VHFQFPADLYEWVRR |
| SEQ ID N. 133 | P280618-02-023 | VHFQFPADLWNWVRR |
| SEQ ID N. 134 | P280618-02-024 | VHFQFPADLWEFVRR |
| SEQ ID N. 135 | P280618-02-025 | VHFQFPADLWEWFRR |
| SEQ ID N. 136 | P280618-02-026 | VHFQFPQDWWEWVRR |
| SEQ ID N. 137 | P280618-02-027 | VHFQIPQDWWEWVRR |
| SEQ ID N. 138 | P280618-02-028 | VHFQFPQDWYEWVRR |
| SEQ ID N. 139 | P280618-02-029 | VHFQFPQDWWNWVRR |
| SEQ ID N. 140 | P280618-02-030 | VHFQFPQDLWEFVRR |
| SEQ ID N. 141 | P280618-02-031 | VHFQFPQDWWEWFRR |
| SEQ ID N. 142 | P280618-02-032 | VHFTIPADFWEWFRR |
| SEQ ID N. 143 | P280618-02-033 | VHVQIPADFWEWFRR |
| SEQ ID N. 144 | P280618-02-034 | VHVTFPADLWEWFRR |
| SEQ ID N. 145 | P280618-02-035 | VHVTIPQDFWEWFRR |
| SEQ ID N. 146 | P280618-02-036 | VHFTIPQDWWEWVRR |
| SEQ ID N. 147 | P280618-02-037 | VHFTFPQDLYNWVRR |
| SEQ ID N. 148 | P280618-02-038 | VHFTFPQDLYNFVRR |
| SEQ ID N. 149 | P280618-02-039 | VHVTIPADLYNFFRR |
| SEQ ID N. 150 | P280618-02-040 | VHFQFPQDLWEWVRR |
| SEQ ID N. 151 | P280618-02-041 | VHFTIPQDLYNWRR |
| SEQ ID N. 152 | P280618-02-042 | VHFTIPADLYNFVRR |
| SEQ ID N. 153 | P280618-02-043 | VHFQIPQDLYNFFRR |
| SEQ ID N. 154 | P280618-02-044 | VHFQFPQEWYNWFRR |
| SEQ ID N. 155 | P280618-02-045 | VRFQFGQEWYNFFRR |
| SEQ ID N. 156 | P280618-02-046 | VPGAGVPGAGIHVTI |
| SEQ ID N. 157 | P280618-02-047 | VPGAGVPGAGIHVTIPA |

The preferred bioconjugate of the present invention are those listed in Table 2.

**Table 2. List of bioconjugate**

| **Bioconjugate name** | **Sequence** |
|---|---|
| Bio-1 | NAM-IMVTIPADLYEWIEE |
| Bio-2 | IMVTIPADLYEWIEEQ-NAG |
| Bio-3 | NAG-NAM-IMVTIPADLYEWIEE |
| Bio-4 | capronic acid- IMVTIPADLYEWIEE |
| Bio-5 | Ascorbic acid - IMVTIPADLYEWIEE |

The preferred Peptide A according to the present invention are those listed in Table 3.

**Table 3. List of the preferred Peptide A.**

| **SEQUENCE ID** | **Sequence** |
|---|---|
| SEQ ID N. 44 | ARKKAAKA |
| SEQ ID N. 45 | TWWTEWSQ |
| SEQ ID N. 46 | TWWETWW |
| SEQ ID N. 47 | VPGWG |
| SEQ ID N. 48 | KETWWETW |
| SEQ ID N. 49 | VPGKG |
| SEQ ID N. 50 | VPGAG |
| SEQ ID N. 51 | RQIKIWFQNRRMKWKK |
| SEQ ID N. 52 | RRRRRRRR |
| SEQ ID N. 53 | VPGDG |
| SEQ ID N. 54 | VPGXG |
| SEQ ID N. 55 | IPGG |
| SEQ ID N. 56 | AVGVP |
| SEQ ID N. 57 | IPGXG |
| SEQ ID N. 58 | IPGVG |
| SEQ ID N. 59 | LPGXG |
| SEQ ID N. 60 | LPGVG |
| SEQ ID N. 61 | VAPGVG |
| SEQ ID N. 62 | XPGVG |
| SEQ ID N. 63 | GVGVPGVG |
| SEQ ID N. 64 | VPGFGVGAG |
| SEQ ID N. 65 | VPGGVPGG |

Wherein in the peptides A of the present invention X= any of the 20 amino acids.

A further object of the present invention is a pharmaceutical composition comprising the aforesaid IL-17A binding polypeptides or bioconjugates and at least one pharmaceutically acceptable excipient.

The pharmaceutical composition of the present invention can be formulated in a form that is suitable for topical or ophthalmic administration.

Preferably, when the administration of the pharmaceutical composition of the invention is performed by topical route, the pharmaceutical form is selected from cream, ointment, gel, salve, solution, washing, suspension, drops, buffer (buffer solution), suspension, eye drops, drops, spray, wipe, or powder, preferably it is selected from cream, gel, spray, or ointment.

Among the ophthalmic administration, the pharmaceutical form is preferably selected from eye drops, ophthalmic gels, ointments, wash, wipe, spray or cream.

According to a particular embodiment the polypeptides or bioconjugates of the present invention are administered locally by using microparticles or nanoparticles.

According to the invention, the pharmaceutical composition of the present invention can be administered to animals and humans, defined as adults and as "paediatric population", wherein with the term "pediatric population "is indicated the part of the population from birth to eighteen years of age.

A further embodiment is the use of the IL-17A binding polypeptide or of the bioconjugate of the present invention in the treatment and/or prevention of inflammatory and autoimmune ophthalmic diseases.

Preferably, said inflammatory and autoimmune ophthalmic diseases are selected from keratoconjunctivitis sicca, vernal keratoconjunctivitis, stromal herpetic keratitis, corneal allograft rejection, corneal infections, such as herpes virus and Pseudomonas aeruginosa keratitis or dry eye disease.

More preferably said ophthalmic diseases is dry eye disease.

A preferred embodiment is the use the polypeptide of formula (II) in the treatment and/or prevention of inflammatory and autoimmune ophthalmic diseases, preferably in the treatment of dry eye disease.

All of the above specified diseases and medical conditions have in common that their origin and/or symptoms are I L-17Aand/or Th-17-related.

More specifically for the latter, dry eye disease (DED), a highly prevalent condition that includes a wide spectrum of ocular surface disorders, ocular mucosal inflammation is a peculiar characteristic potentially leading to vision loss if uncontrolled, due to inflammation-induced corneal ulceration and scarring. In the progression of DED, pathogenic immune cells, predominantly Th17 cells continuously migrate to the ocular mucosal surface and secrete pro-inflammatory mediators, including IL17, causing ocular surface inflammation and epitheliopathy.

A further embodiment of the present invention is the use of one the polypeptides listed in Table 4 in the treatment and/or prevention of inflammatory and autoimmune ophthalmic diseases.

Preferably, said inflammatory and autoimmune ophthalmic diseases are selected from keratoconjunctivitis sicca, vernal keratoconjunctivitis, stromal herpetic keratitis, corneal allograft rejection, corneal infections, such as herpes virus and Pseudomonas aeruginosa keratitis and dry eye disease.

More preferably said ophthalmic diseases is dry eye disease.

**Table 4.**

| **SEQ ID N.** | **SEQUENCE** |
|---|---|
| SEQ ID N. 1 | IHVTIPADLWDWINK |
| SEQ ID N. 66 | IVVTMPADLWDWIKA |
| SEQ ID N. 67 | IVVTMPADLWDWIRA |
| SEQ ID N. 68 | IVVTMPADLWDWIRK |
| SEQ ID N. 69 | IVVTMPADLWDWIAA |
| SEQ ID N. 70 | IVVTMPADLWDWARA |
| SEQ ID N. 71 | IVVTMPADLWAWIRA |
| SEQ ID N. 72 | IVVTMPADLADWIRA |
| SEQ ID N. 73 | IVVTMPADAWDWIRA |
| SEQ ID N. 74 | IVVTMPADLWDWIRA |
| SEQ ID N. 75 | IVVTAPADLWDWIRA |
| SEQ ID N. 76 | IVVAMPADLWDWIRA |
| SEQ ID N. 77 | IAVTMPADLWDWIRA |
| SEQ ID N. 78 | AVVTMPADLWDWIRA |
| SEQ ID N. 79 | IHVTMPADLWDWIRA |
| SEQ ID N. 80 | IQVTMPADLWDWIRA |
| SEQ ID N. 81 | IRVTMPADLWDWIRA |
| SEQ ID N. 82 | ITVTMPADLWDWIRA |
| SEQ ID N. 83 | IWVTMPADLWDWIRA |
| SEQ ID N. 84 | IYVTMPADLWDWIRA |
| SEQ ID N. 85 | IVVTIPADLWDWIRA |
| SEQ ID N. 86 | IVVTLPADLWDWIRA |
| SEQ ID N. 87 | IVVTVPADLWDWIRA |
| SEQ ID N. 88 | IVVTMPADLWDWIMA |
| SEQ ID N. 89 | IVVTMPADLWDWINA |
| SEQ ID N. 90 | IVVTMPADLWDWIQA |
| SEQ ID N. 91 | IVVTIPADLWDWIRA |
| SEQ ID N. 92 | IVVTLPADLWDWIRA |
| SEQ ID N. 93 | IHVTIPADLWDWINK |
| SEQ ID N. 94 | IHVTIPADLWDWIN |
| SEQ ID N. 95 | IHVTIPADLWDWI |
| SEQ ID N. 96 | DSSAVCWAFPHHPLCHMKAT |
| SEQ ID N. 97 | ADADMCWFFPTSPWCH |
| SEQ ID N. 98 | DLSAVCWAFPWDPECHM |
| SEQ ID N. 99 | DSSAVCWAFPYLPECH |
| SEQ ID N. 100 | DISAVCWAFPFDPECH |
| SEQ ID N. 101 | AYECPRLEYDMFGALHCLPS |
| SEQ ID N. 102 | CPRLEYDMFGALHCL |
| SEQ ID N. 103 | CLDLQYDPWGALHCI |
| SEQ ID N. 104 | CFDLQYDPWGALHCI |
| SEQ ID N. 105 | CLDLQYDMFGALHCV |
| SEQ ID N. 106 | CLDLVYDPWGALHCI |
| SEQ ID N. 107 | CWVLEYDMFGALHCR |
| SEQ ID N. 108 | CWALEYDMFGYLHCR |
| SEQ ID N. 109 | CWVLEYDMFGFLHCR |
| SEQ ID N. 110 | CWVLEYDMFGYLHCR |

The amount and mode of administration of the polypeptides according to the present invention will vary depending upon the particular polypeptide inhibitor of the invention, the individual patient group or patient, the presence of further medically active compounds and the nature and severity of the condition being treated.

According to a preferred embodiment, the prophylactic and/or therapeutic use dosages is of about 5-50 µg/ml, preferably of about 10-25 µg/ml

Preferably, the frequency of administration for prophylactic and/or therapeutic uses lies in the range of about once to twice daily applications, preferably once.

IL-17A-binding polypeptides or conjugate of the invention are conveniently and preferably administered topically as eye drops or ophthalmic gels and ointments.

In a further embodiment the IL-17A binding polypeptide or a bioconjugate for use according to the present invention, it is administered as the only active principle or in combination with further active principles, and/or in combination with medical devices for the symptomatic treatment of ophthalmic conditions including but not limited to DED, e.g. ocular lubricants or "artificial tears", topical re-epithelizing agents, therapeutic contacts lenses and punctum plugs.

Preferably, said further active principle is an adjuvant, immunosuppressive agent, immunomodulating agent or anti-inflammatory agent.

For example, the IL-17A binding polypeptide of the present invention may be used in combination with DMSO.

According to a preferred embodiment, the IL-17A binding polypeptide of the present invention may be used in combination with immunosuppressive monoclonal antibodies, such as monoclonal antibodies with affinity to leukocyte receptors, selected from MHC, CD2, CD3, CD4, CD7, CD8, CD25, CD28, CD40, CD45, CD58, CD80, CD86 or their ligands; other immunomodulatory compounds, preferably a recombinant binding molecule having at least a portion of the extracellular domain of CTLA4 or a mutant thereof, an at least extracellular portion of CTLA4 or a mutant thereof joined to a non-CTLA4 protein sequence, e.g. (e.g. CTLA4Ig, designated ATCC 68629) or a mutant thereof, e.g. LEA29Y; adhesion molecule inhibitors, LFA-I antagonists, ICAM-I or -3 antagonists, VCAM-4 antagonists or VLA-4 antagonists.

In a further preferred embodiment the IL-17A binding polypeptide of the present invention is used in combination with DMARD (disease-modifying antirheumatic drug), preferably Gold salts, sulphasalazine, anti- malarias, methotrexate, D-penicillamine, azathioprine, mycophenolic acid, cyclosporine A, tacrolimus, sirolimus, minocycline, leflunomide, glucocorticoids; a calcineurin inhibitor, preferably cyclosporin A or FK 506; a modulator of lymphocyte recirculation, preferably FTY720 and FTY720 analogs; a mTOR inhibitor, preferably rapamycin, 40-O-(2- hydroxyethyl)-rapamycin, CCI779, ABT578, AP23573 or TAFA-93; an ascomycin having immuno-suppressive properties, preferably ABT-281, ASM981; corticosteroids; cyclophosphamide; azathioprene; methotrexate; leflunomide; mizoribine; mycophenolic acid; mycophenolate mofetil; 15-deoxyspergualine or an immunosuppressive homologue, analogue or derivative thereof; or a chemotherapeutic agent, preferably pacli- taxel, gemcitabine, cisplatinum, doxorubicin or 5-fluorouracil; anti-TNF agents, preferably monoclonal antibodies to TNF, preferably infliximab, adaiimumab, CDP870, or receptor constructs to TNF-RI or TNF-RII, preferably Etanercept, PEG-TNF-RI; blockers of proinflammatory cytokines, IL-1 blockers, preferably Anakinra or IL-1 trap, AAL160, ACZ 885, IL- 6 blockers; inhibitors or activators of proteases, preferably metalloproteases, anti-IL-15 antibodies, anti-IL-6 antibodies, anti-IL-23 antibodies, anti-IL-22 antibodies, anti-IL-21 antibodies, anti-IL-12 antibodies, anti-IFN-gamma antibodies, anti-IFN-alpha antibodies, anti-CD20 antibodies, anti-inflammatory agents, preferably aspirin or an anti-infectious agent.

Naturally, this list of agents for co-administration is not limiting nor complete.

### Material and methods

The invention is further described by way of illustration in the following examples, none of which are to be interpreted as limiting the scope of the invention as outlined in the appended claims.

### Experimental procedures

### Peptide synthesis & purification

We've developed an homology model of IL17A by the use of crystallographic data available (PDB 5h13, PDB 5vb9 and PDB 4hsa)(Liu, Song et al. 2013, Liu, Dakin et al. 2016, Liu, Desharnais et al. 2016, Ting, Tung et al. 2018), in our models we've also reconstructed the loops unresolved in the crystals and we optimized models through a molecular dynamics simulation with Desmond software as implemented in Schrodinger Maestro macromodel suite. All molecular simulations have been run for 1 microsec to guarantee the stability of the system. Basing on the structure and properties of the α-pocket on IL17A different peptidic sequences have been designed to have a stable secondary and tertiary conformations to engage the IL17A with an optimized receptor occupancy and to have suitable phys-chem properties for the topical use in the eye pathologies (Liu, Song et al. 2013, Espada, Broughton et al. 2016). Additionally, we've optimized the interactions ligand-IL17A by the introduction of H bonds and optimization of hydrophobic interactions.

### Fmoc-Based Solid-Phase Peptide Synthesis

All the chemicals were purchased from main chemical suppliers and used without further purification. All the peptides were prepared by manual or automatic solid-phase peptide synthesis, with Rink Amide-MBHA resin as the solid support. Fmoc groups for Nα-protection were cleaved by 8 min treatment with 20% piperidine in DMF followed by the second treatment with the same reagent for 10 min. After the Fmoc cleavage, the peptide-resin was washed with DMF (× 6). The next residue was then incorporated with the HATU/DIPEA coupling protocol [Fmoc-amino acid (3 equiv), HATU (3 equiv), and DIPEA (6 equiv)]. After gentle agitation (1 hr) and washing with DMF (× 6), part of the peptide-resin was analyzed by Kaiser test. On completion of the assembly, the peptide-resin was successively washed with DMF (× 3), DCM (× 4) and then dried in vacuo.

### Cleavage and Deprotection

The cleavage mixture (Trifluoroacetic acid/1, 2-ethanedithiol/ thioanisole/phenol/H2O/triisopropylsilane = 68.5/10/10/5/3.5/1, v/v, 3 ml/100 mg of resin).

Complete deprotection was achieved in the mixture in 4 hrs at 30C. Following the cleavage reaction, the peptide in the mixture was precipitated in cold ether and dried in vacuo at 50C.

### Disulfide Bond Formation

Disulfide formation of the peptide Lot No.: P240418-01-01 (see table 1) was made in the mixture of DMSO/H2O (20/80, v/v), which was monitored by RP-HPLC, MS and free sulfhydryl detection (DTNB method).

### Purification by Preparative RP-HPLC and Purity Assessment by Analytical HPLC

The crude peptide samples were purified by preparative RP-HPLC (Agilent) using a column of C18 (10 µm, 100 Å, 50 × 250 mm). A solvent system consisting of solvent A (0.1 % TFA, 2 % CH3CN in water) and solvent B (90 % CH3CN/H2O) at a flow rate of 25 mL/min was used for elution, and the absorbance was detected at 220 nm. The solvent was removed by lyophilization. The final products were characterized by MALDI-TOF-MS, and the purity of the purified material was assessed by analytical RP-HPLC (C18-250 mm × 4.6 mm I.D., flow rate of 1 mL/min), and the absorbance was detected at 220 nm.

### IL17RA/IL17RC dimerization assay in response to IL-17A

Interleukin receptor RA and RC dimerization assay (DiscoverX) uses a dimerization approach to measure interaction of the receptor chains upon activation. In the PathHunter® Cytokine Receptor Assay, one cytokine receptor chain is tagged with a small peptide epitope ProLink (PK) and the other chain is tagged with Enzyme Acceptor (EA). Ligand binding induces dimerization of the two receptors, facilitating complementation of PK and EA fragments. This interaction generates an active unit of b-galactosidase, which is detected using a chemiluminescent substrate. The method is ideal for investigating the activity of biologics and antibody therapeutics that activate or block receptor dimerization by either binding to the target or ligand. The dimerization is observed in presence of IL17A and can be disrupted by IL17A binding peptides. PathHunter cell lines were expanded from freezer stocks according to standard procedures. Cells were seeded in a total volume of 20 µL into white walled, 384-well microplates and incubated for the appropriate time prior to testing. For agonist determination, cells were incubated with sample to induce response. Intermediate dilution of sample stocks was performed to generate 5X sample in assay buffer. 5 µL of 5X sample was added to cells and incubated at 37°C or room temperature for 60 minutes. Vehicle concentration was 1%. 5 µL of 6X EC80 agonist in assay buffer was added to the cells and incubated at 37°C for 6 to 16 hours depending on the assay. 1. Assay signal was generated through a single addition of 12.5 or 15 µL (50 % v/v) of PathHunter Detection reagent cocktail for agonist and antagonist assays respectively, followed by a one hour incubation at room temperature. For some assays, activity was detected using a high sensitivity detection reagent (PathHunter Flash Kit) to improve assay performance. In these assays, an equal volume of detection reagent (25 or 30uL) was added to the wells, followed by a one hour incubation at room temperature. Microplates were read following signal generation with a PerkinElmer EnvisionTM instrument for chemiluminescent signal detection. Compound activity was analyzed using CBIS data analysis suite (Chemlnnovation, CA). % Inhibition =100% x (1 - (mean RLU of test sample - mean RLU of vehicle control) / (mean RLU of EC80 control - mean RLU of vehicle control)).

### Phenotipic assay in human differentiated TH17 cells and viability assay

Human primary CD4 T-cells were differentiated into Th17 cells in vitro by culturing the cells in Th17 differentiation medium for 10 days. Following differentiation, the cells were stimulated with either an activation cocktail containing Phorbol 12-myristate 13-acetate and lonomycin in the presence of varying concentrations of test articles. Following stimulation (timing 24h), culture supernatants were analyzed for expression of IL-17, IL-6, IL-23, MMP3 and examined for viability using the fluorescence viability dye, Alamar blue. The CD4 cells secreted high levels of IL-17A in response to stimulation with an activation cocktail, indicating successful differentiation into Th17 cells. Additionally, stimulated cells secreted IL-6, IL23 and MMP3.

Purified human peripheral blood CD4 T-cells were cultured in Th-17 differentiation medium (CellXVivo Human Th17, Tocris) per manufacturer's instructions. Briefly: 96-well tissue culture plates were coated with anti-CD3 and CD28 agonistic antibodies. Cells were counted via trypan blue exclusion and suspended at a concentration of 1E5 cells/ml in Th-17 differentiation medium. 0.2 mL of this suspension was plated into individual wells of a 96-well plate. Cells were incubated at 37°C with 5% CO2 for 10 days, with media replaced as needed. Following 10-day Cell Differentiation, test items, such as P260318-01-19, were diluted to the appropriate concentration in CGM (cell growth medium, XVivo-15, Lonza). Cell plate was centrifuged at 300xg for 5 min, and Th17 differentiation medium was removed. Supernatant was removed and replaced with 200 µL of diluted test items or vehicle (CGM + DMSO diluted as with TI concentration) in appropriate groups. Cells were incubated with test items for 1 hour before stimulation. The following stimulation solutions were generated: 10x Cell Activation Cocktail: Diluted from 500x to 10x working solution in CGM. 22 µL of appropriate stimulants were added to wells 1 hr after TI addition. Finally, 25 µL of Alamar blue reagent (10x) was added to each well 20 hours after stimulant (4 hrs prior to 24 hr serum collection) to determine cell viability. 24 hrs After Stimulant, Alamar blue fluorescence was measured per manufacturer's instructions. Remaining Supernatants were collected for subsequent multiplex protein analyses. Expression levels of IL-6, IL-17, and IL-23 as well as MMP3 in 24 hour stimulated culture supernatants was evaluated using a Luminex Multiplex Platform on a MAGPIX instrument.

### Results

### IL17RA/IL17RC dimerization assay in response to IL-17A

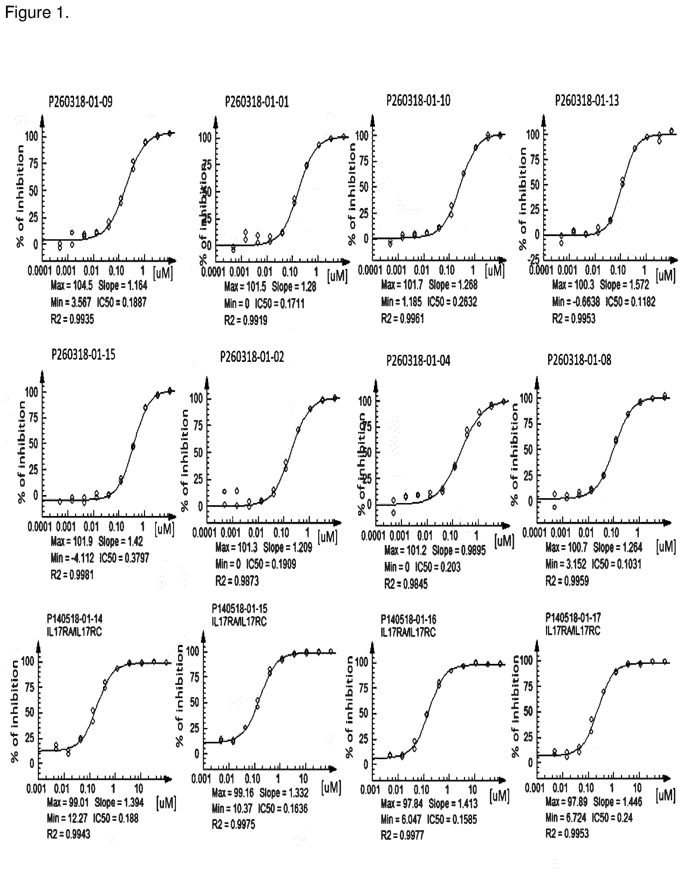

The dimerization approach has been used to measure interaction of the receptor chains IL17RA and IL17Rc upon activation by IL17A. In the PathHunter® Cytokine Receptor Assay, one cytokine receptor chain is tagged with a small peptide epitope ProLink (PK) and the other chain is tagged with Enzyme Acceptor (EA). Ligand binding induces dimerization of the two receptors, facilitating complementation of PK and EA fragments. This interaction generates an active unit of b-galactosidase, which is detected using a chemiluminescent substrate. This method is ideal for investigating the activity of compounds that block receptor dimerization by either binding to the target or ligand. We've tested our compounds primary activity in this assay thus evaluating their ability to inhibit IL17RA/IL17RC dimerization. As reported in table 5, the sequences selected and tested in IL17RA/IL17RC dimerization assay resulted active with a IC50 in the mid nanomolar range. In figure 1, as example, the dose-response of different peptides is reported. Tested peptidic sequences are able to inhibit IL17RA/IL17RC dimerization thus inhibiting the IL17A pathway activation.

### Phenotipic assay in human differentiated TH17 cells and viability assay

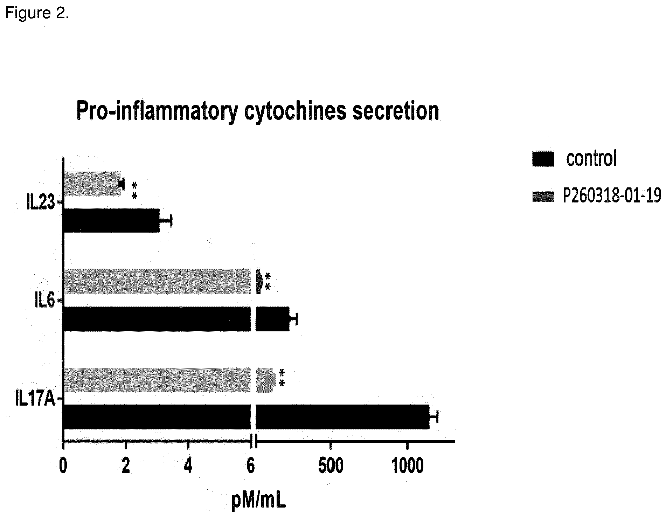

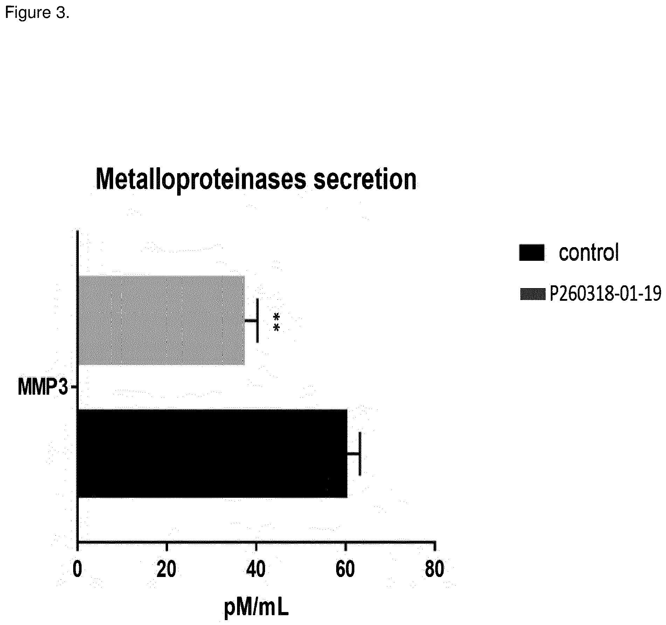

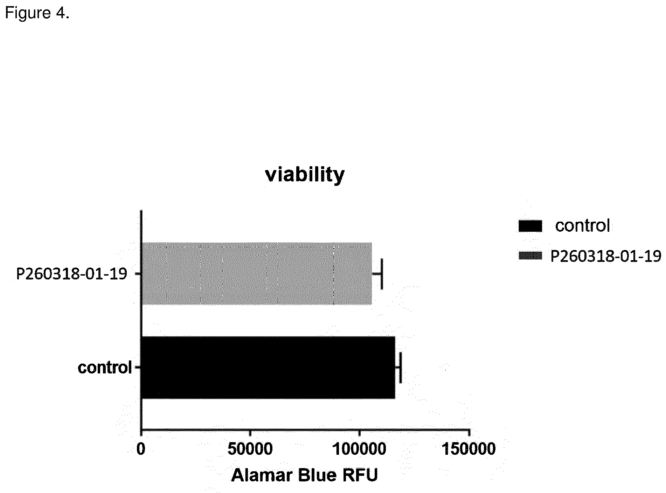

To evaluate the effect of our peptides on the target cells responsible for inflammation and subsequent damage in ocular pathologies as dry eye, we observed the ability of our peptides to inhibit pro-inflammatory cytokines and metalloproteinases secretion in human Th17 cells in vitro. As an example, figures 2 and 3 the effect of P260318-01-19 peptide is reported. P260318-01-19 sequence is able to significantly reduce the secretion of IL-17A, IL6 and IL23 as well as it reduces the secretion of MMP3. As reported in figure 4 the inhibition of cytokines and metalloproteinases secretion is not due to cell viability issues since cell treatment with P260318-01-19 for eleven days does not impair cell viability.

**Table 5. Preferred sequences activity in IL17RA/IL17RC dimerization assay.**

| **SEQ ID N.** | **Peptide/bioconjugate name** | **IC50 in IL17RA/IL7RC dimerization assay** |
|---|---|---|
| SEQ ID N. 1 | P260318-01-01 | <0.2 µM |
| SEQ ID N. 2 | P260318-01-02 | <0.2 µM |
| SEQ ID N. 3 | P260318-01-03 | <0.4 µM |
| SEQ ID N. 4 | P260318-01-04 | <0.1 µM |
| SEQ ID N. 5 | P260318-01-05 | <0.1 µM |
| SEQ ID N. 6 | P260318-01-07 | <0.4 µM |
| SEQ ID N. 7 | P260318-01-08 | <0.1 µM |
| SEQ ID N. 8 | P260318-01-09 | <0.2 µM |
| SEQ ID N. 9 | P260318-01-10 | <0.3 µM |
| SEQ ID N. 10 | P260318-01-11 | <0.4 µM |
| SEQ ID N. 11 | P260318-01-12 | <0.4 µM |
| SEQ ID N. 12 | P260318-01-13 | <0.2 µM |
| SEQ ID N. 13 | P260318-01-14 | <0.4 µM |
| SEQ ID N. 14 | P260318-01-15 | <0.4 µM |
| SEQ ID N. 15 | P260318-01-16 | <0.4 µM |
| SEQ ID N. 16 | P260318-01-17 | <0.4 µM |
| SEQ ID N. 17 | P260318-01-18 | <0.4 µM |
| SEQ ID N. 18 | P260318-01-19 | <0.4 µM |
| SEQ ID N. 19 | P240418-01-02 | < 1 µM |
| SEQ ID N. 20 | P240418-01-03 | < 1 µM |
| SEQ ID N. 21 | P240418-01-04 | < 1 µM |
| SEQ ID N. 22 | P240418-01-05 | < 1 µM |
| SEQ ID N.23 | P240418-01-06 | < 1 µM |
| SEQ ID N. 24 | P240418-01-07 | < 1 µM |
| SEQ ID N. 25 | P140518-01-01 | < 1 µM |
| SEQ ID N. 26 | P140518-01-02 | <1 µM |
| SEQ ID N. 27 | P140518-01-03 | <1 µM |
| SEQ ID N. 28 | P140518-01-05 | <1 µM |
| SEQ ID N. 29 | P140518-01-06 | <1 µM |
| SEQ ID N. 30 | P140518-01-07 | < 1 µM |
| SEQ ID N. 31 | P140518-01-08 | < 1 µM |
| SEQ ID N. 32 | P140518-01-09 | < 1 µM |
| SEQ ID N. 33 | P140518-01-10 | < 1 µM |
| SEQ ID N. 34 | P140518-01-11 | < 1 µM |
| SEQ ID N. 35 | P140518-01-12 | < 1 µM |
| SEQ ID N. 36 | P140518-01-13 | < 1 µM |
| SEQ ID N. 37 | P140518-01-14 | < 0.2 µM |
| SEQ ID N. 38 | P140518-01-15 | < 0.2 µM |
| SEQ ID N. 39 | P140518-01-16 | < 0.2 µM |
| SEQ ID N. 40 | P140518-01-17 | < 0.2 µM |
| SEQ ID N. 41 | P240418-01-01 | < 1 µM |
| SEQ ID N. 111 | P280618-02-01 | <0.5 µM |
| SEQ ID N. 112 | P280618-02-02 | <0.5 µM |
| SEQ ID N. 113 | P280618-02-03 | <0.5 µM |
| SEQ ID N. 114 | P280618-02-04 | <0.5 µM |
| SEQ ID N. 115 | P280618-02-05 | <0.5 µM |
| SEQ ID N. 116 | P280618-02-06 | <0.5 µM |
| SEQ ID N. 117 | P280618-02-07 | <0.5 µM |
| SEQ ID N. 118 | P280618-02-08 | <0.5 µM |
| SEQ ID N. 119 | P280618-02-09 | <0.5 µM |
| SEQ ID N. 120 | P280618-02-010 | <0.5 µM |
| SEQ ID N. 121 | P280618-02-011 | <0.5 |
| SEQ ID N. 122 | P280618-02-012 | <0.5 µM |
| SEQ ID N. 123 | P280618-02-013 | <0.5 µM |
| SEQ ID N. 124 | P280618-02-014 | <0.5 µM |
| SEQ ID N. 125 | P280618-02-015 | <0.5 µM |
| SEQ ID N. 126 | P280618-02-016 | <0.5 µM |
| SEQ ID N. 127 | P280618-02-017 | <0.5 µM |
| SEQ ID N. 128 | P280618-02-018 | <0.5 µM |
| SEQ ID N. 129 | P280618-02-019 | <0.5 µM |
| SEQ ID N. 130 | P280618-02-020 | <0.5 µM |
| SEQ ID N. 131 | P280618-02-021 | <0.5 µM |
| SEQ ID N. 132 | P280618-02-022 | <0.5 µM |
| SEQ ID N. 133 | P280618-02-023 | <0.5 µM |
| SEQ ID N. 134 | P280618-02-024 | <0.5 µM |
| SEQ ID N. 135 | P280618-02-025 | <0.5 µM |
| SEQ ID N. 136 | P280618-02-026 | <0.5 µM |
| SEQ ID N. 137 | P280618-02-027 | <0.5 µM |
| SEQ ID N. 138 | P280618-02-028 | <0.5 µM |
| SEQ ID N. 139 | P280618-02-029 | <0.5 µM |
| SEQ ID N. 140 | P280618-02-030 | <0.5 µM |
| SEQ ID N. 141 | P280618-02-031 | <0.5 µM |
| SEQ ID N. 142 | P280618-02-032 | <0.5 µM |
| SEQ ID N. 143 | P280618-02-033 | <0.5 µM |
| SEQ ID N. 144 | P280618-02-034 | <0.5 µM |
| SEQ ID N. 145 | P280618-02-035 | <0.5 µM |
| SEQ ID N. 146 | P280618-02-036 | <0.5 µM |
| SEQ ID N. 147 | P280618-02-037 | <0.5 µM |
| SEQ ID N. 148 | P280618-02-038 | <0.5 µM |
| SEQ ID N. 149 | P280618-02-039 | <0.5 µM |
| SEQ ID N. 150 | P280618-02-040 | <0.5 µM |
| SEQ ID N. 151 | P280618-02-041 | <0.5 µM |
| SEQ ID N. 152 | P280618-02-042 | <0.5 µM |
| SEQ ID N. 153 | P280618-02-043 | <0.5 µM |
| SEQ ID N. 154 | P280618-02-044 | <0.5 µM |
| SEQ ID N. 155 | P280618-02-045 | <0.5 µM |
| SEQ ID N. 156 | P280618-02-046 | <0.5 µM |
| SEQ ID N. 157 | P280618-02-047 | <0.5 µM |
| | Bio-1 | < 1 µM |
| | Bio-2 | < 1 µM |
| | Bio-3 | < 1 µM |
| | Bio-4 | < 1 µM |
| | Bio-5 | < 1 µM |

### In vivo test on the efficacy of the peptides.

The *in vivo* model to evaluate the efficacy of our peptides is an established murine model of dry eye. In the proposed model dry eye disease is induced by a transdermic application of a scopolamine patch and by the exposure of the animals to low humidity conditions (<25%). The evaluation of dry eye development and of compounds efficacy is conducted after fifteen days by fluorescein instillation and slit lamp examination of the eyes to observe superficial punctuate keratitis grade. Additionally tear secretion by phenol red thread tear test could be evaluated and goblet cells state or inflammatory cells presence could be determined by hystologic examination of the ocular tissues (Barabino, Shen et al. 2005).

### References

- Barabino, S., L. Shen, L. Chen, S. Rashid, M. Rolando and M. R. Dana (2005). "The controlled-environment chamber: a new mouse model of dry eye." Invest Ophthalmol Vis Sci 46(8): 2766-2771.
- Beringer, A., M. Noack and P. Miossec (2016). "IL-17 in Chronic Inflammation: From Discovery to Targeting." Trends Mol Med 22(3): 230-241.
- Chauhan, S. K., J. El Annan, T. Ecoiffier, S. Goyal, Q. Zhang, D. R. Saban and R. Dana (2009). "Autoimmunity in Dry Eye Is Due to Resistance of Th17 to Treg Suppression." The Journal of Immunology 182(3): 1247-1252.
- Chen, Y., S. K. Chauhan, H. S. Lee, D. R. Saban and R. Dana (2014). "Chronic dry eye disease is principally mediated by effector memory Th17 cells." Mucosal Immunol 7(1): 38-45.
- De Paiva, C. S., S. Chotikavanich, S. B. Pangelinan, J. D. Pitcher, 3rd, B. Fang, X. Zheng, P. Ma, W. J. Farley, K. F. Siemasko, J. Y. Niederkorn, M. E. Stern, D. Q. Li and S. C. Pflugfelder (2009). "IL-17 disrupts corneal barrier following desiccating stress." Mucosal Immunol 2(3): 243-253.
- de Paiva, C. S., A. J. W. Huang, D.-Q. Li and S. C. Pflugfelder (2014). "New Understandings on Pathogenesis of Dry Eye - From Animal Models to Clinical Therapy."
- Espada, A., H. Broughton, S. Jones, M. J. Chalmers and J. A. Dodge (2016). "A Binding Site on IL-17A for Inhibitory Macrocycles Revealed by Hydrogen/Deuterium Exchange Mass Spectrometry." J Med Chem 59(5): 2255-2260.
- Gu, C., L. Wu and X. Li (2013). "IL-17 family: cytokines, receptors and signaling." Cytokine 64(2): 477-485.
- Hah, Y. S., H. J. Chung, S. B. Sontakke, I. Y. Chung, S. Ju, S. W. Seo, J. M. Yoo and S. J. Kim (2017). "Ascorbic acid concentrations in aqueous humor after systemic vitamin C supplementation in patients with cataract: pilot study." BMC Ophthalmol 17(1): 121.
- Liu, S., L. A. Dakin, L. Xing, J. M. Withka, P. V. Sahasrabudhe, W. Li, M. E. Banker, P. Balbo, S. Shanker, B. A. Chrunyk, Z. Guo, J. M. Chen, J. A. Young, G. Bai, J. T. Starr, S. W. Wright, J. Bussenius, S. Tan, A. Gopalsamy, B. A. Lefker, F. Vincent, L. H. Jones, H. Xu, L. R. Hoth, K. F. Geoghegan, X. Qiu, M. E. Bunnage and A. Thorarensen (2016). "Binding site elucidation and structure guided design of macrocyclic IL-17A antagonists." Sci Rep 6: 30859.
- Liu, S., J. Desharnais, P. V. Sahasrabudhe, P. Jin, W. Li, B. D. Oates, S. Shanker, M. E. Banker, B. A. Chrunyk, X. Song, X. Feng, M. Griffor, J. Jimenez, G. Chen, D. Tumelty, A. Bhat, C. W. Bradshaw, G. Woodnutt, R. W. Lappe, A. Thorarensen, X. Qiu, J. M. Withka and L. D. Wood (2016). "Inhibiting complex IL-17A and IL-17RA interactions with a linear peptide." Sci Rep 6: 26071.
- Liu, S., X. Song, B. A. Chrunyk, S. Shanker, L. R. Hoth, E. S. Marr and M. C. Griffor (2013). "Crystal structures of interleukin 17A and its complex with IL-17 receptor A." Nat Commun 4: 1888.
- Ma, N., C. Siegfried, M. Kubota, J. Huang, Y. Liu, M. Liu, B. Dana, A. Huang, D. Beebe, H. Yan and Y. B. Shui (2016). "Expression Profiling of Ascorbic Acid-Related Transporters in Human and Mouse Eyes." Invest Ophthalmol Vis Sci 57(7): 3440-3450.
- Onishi, R. M. and S. L. Gaffen (2010). "Interleukin-17 and its target genes: mechanisms of interleukin-17 function in disease." Immunology 129(3): 311-321.
- Parul Singh, P. S. "Role of Topical Ascorbic Acid in Management of Refractory Corneal Ulcer." Journal of Pharmacy(6): 2319-4219.
- Parul Singh, P. S. (2012). "Role of Topical Ascorbic Acid in Management of refractory corneal ulcer." Journal of pharmacy 2(6): 01-04.
- Rowe, R. C., P. J. Sheskey, W. G. Cook, M. E. Fenton and American Pharmacists Association. (2012). Handbook of pharmaceutical excipients / edited by Raymond C. Rowe, BPharm, PhD, DSC, FRPharmS, FRSC, CPhys, MInstP, chief scientist, Paul J. Sheskey, BSc, RPh, principal research scientist, the Dow Chemical Company, Midland, MI, USA, Walter G. Cook, BSc, PhD, research fellow, Materials Science group of Pharmaceutical R&D, Pfizer, Sandwich, Kent, UK, Marian E. Fenton, BSc, MSc, development editor, Royal Pharmaceutical Society of Great Britain, London, UK. London, APhA/Pharmaceutical Press.
- Simon French, B. R. (1983). "What is a conservative substitution?" journal of molecular evolution 19(2): 171-175.
- Stern, M. E., C. S. Schaumburg, R. Dana, M. Calonge, J. Y. Niederkorn and S. C. Pflugfelder (2010). "Autoimmunity at the ocular surface: pathogenesis and regulation." Mucosal Immunol 3(5): 425-442.
- Stevenson, W., S. K. Chauhan and R. Dana (2012). "Dry eye disease: an immune-mediated ocular surface disorder." Arch Ophthalmol 130(1): 90-100.
- Subbarayal, B., S. K. Chauhan, A. Di Zazzo and R. Dana (2016). "IL-17 Augments B Cell Activation in Ocular Surface Autoimmunity." J Immunol 197(9): 3464-3470.
- Ting, J. P., F. Tung, S. Antonysamy, S. Wasserman, S. B. Jones, F. F. Zhang, A. Espada, H. Broughton, M. J. Chalmers, M. E. Woodman, H. A. Bina, J. A. Dodge, J. Benach, A. Zhang, C. Groshong, D. Manglicmot, M. Russell and S. Afshar (2018). "Utilization of peptide phage display to investigate hotspots on IL-17A and what it means for drug discovery." PLoS One 13(1): e0190850.

## Claims

1. A IL-17A binding polypeptide, inhibitor of the complex IL17A/IL17RA/IL17RC interaction, having either formula:
(I) X₁-X₂-X₃-X₄-X₅-X₆-X₇-X₈-X₉-X₁₀-X₁₁-X₁₂-X₁₃-X₁₄-X₁₅
wherein, independently from each other:
X₁ = I, V, D, K or W;
X₂= H, M, K or N
X₃ = V or F;
X₄ = T, Q, H or Y;
X₅ = I or F;
X₆ = P or G;
X₇ = A or Q;
X₈ = D , E or N;
X₉ = L, V, F or W;
X₁₀ = W, Y, F or absent;
X₁₁= D, E, N or absent;
X₁₂ = W, F, V or absent;
X₁₃ = I, V, F or absent;
X₁₄ = N, R, E, F or absent;
X₁₅ = K, R, E, F, V, W or absent;
and with the proviso that the formula is not IHVTIPVDLWDWINK;
or formula:
(II) aₙ-DLSAVCWAFPWDPECH-b_{n'}
wherein, independently from each other:
a or b = alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine or valine;
n = 0 to 3;
n' = 0 to 3;
and each of the amino acid different from a or b can be replaced with a conservative substitution.

2. A IL-17 binding polypeptide, inhibitor of the complex IL17A/IL17RA/IL17RC interaction, according to claim 1, **characterized in that** said polypeptide has an amino acid sequence with at least 80% of identity to the sequence of formula (I) or (II), more preferably at least 85-90% of identity,

3. A IL-17A binding polypeptide, inhibitor of the complex IL17A/IL17RA/L17RC interaction, according to claim 1, wherein in the polypeptide of formula (I):
X₁ = I or V;
X₂= H or M;
X₃ = V;
X₄ = T;
X₅ = I;
X₆ = P;
X₇ = A;
X₈ = D;
X₉ = L;
X₁₀ = W or Y;
X₁₁= D or E;
X₁₂ = W;
X₁₃ = I or V;
X₁₄ = N, R or E;
X₁₅ = K, R or E;

4. A bioconjugate comprising the IL-17A binding polypeptide sequence of formula (I) or (II) according to claim 1, having the following formula:
(III) [Biomolecule or CAP]ₐ - [Linker]_{b} - (Sequence A)ₙ-Aₘ-[Peptide] - A'_{m'}-(Sequence A)_{n'} -[Linker]_{b'} - [Biomolecule or CAP]_{a'}
wherein, independently from each other:
a = 0 or 1;
b = 0 or 1 ;
a' = 0 or 1;
b' = 0 or 1;
m = 0 to 10;
m' = 0 to 10;
n= 0 or 5;
n'= 0 or 5,
and
Peptide is the polypeptide according to formula (I) or formula (II);
A or A'= one amino acid selected from Arg, Lys, Gly, Glu or Ala repeated m or m' times;
Sequence A comprises the sequence of formula:
(IV) - Y₁-Y₂-Y₃-Y₄-Y₅-Y₆-Y₇-Y₈-Y₉-Y₁₀-Y₁₁-Y₁₂-Y₁₃-Y₁₄-Y₁₅-Y₁₆-
wherein:
Y₁= A, T, V, K, R, I, L, X or G;
Y₂= R, W, P, E, Q or A;
Y₃= K, W, G, T, I, R or P;
Y₄= K, T, E, W, A, R, D, G, X or F;
Y₅= A, E, T, G, W, I, R, P or V;
Y₆= A, W, E, R, G, P or is absent;
Y₇=K, S, W, T, F, R, V, G or is absent;
Y₈= A, Q, W, R, G or is absent;
Y₉ = N, G or is absent;
Y₁₀ = R or is absent;
Y₁₁ = R or is absent;
Y₁₂ = M or is absent;
Y₁₃ = K or is absent;
Y₁₄ = W or is absent;
Y₁₅ = K or is absent;
Y₁₆ = K or is absent;
and
Biomolecule is, independently from each other, capronic acid, ascorbic acid, NAG-NAM, NAG, NAM, hyaluronic acid, alginic acid, chitin, (GalNAc)₂, Gal-alpha1,3-GalNAc or trigalacturonic acid;
Cap is, independently from each other, amide, aldehyde, ester, p-Nitroanilide, 7-Amino-4-Methylcoumarin, acetyl, formyl, pyroglutamyl or a fatty acid;
Linker is, independently from each other, 4-aminobutyric acid, beta-alaninine, 2-aminoethoxy-acetic acid, 5-aminovaleric acid, 6-aminocaproic acid, 8-Amino-3,6-dioxaoctanoic acid, 12-amino-4,7,10-trioxadodecanoic acid, 15-amino-4,7,10,13-tetraoxapenta-decanoic acid or trioxatridecan-succinamic acid.

5. A bioconjugate according to claim 4, **characterized in that** Sequence A is attached to the N-terminal or to the C-terminal of the peptide of formula (I) or (II).

6. A bioconjugate according to claim 4, **characterized in that** the biomolecule is selected from ascorbic acid, NAG or NAM.

7. A bioconjugate according to claim 4, **characterized in that** the Cap is a a C-ter modification selected from amides, preferably N-alkyl amides, aldehydes, esters, preferably methyl and ethyl esters, p-Nitroanilide, 7-Amino-4-Methylcoumarin or a N-ter modification selected from acetyl, formyl, pyroglutamyl, fatty acids, preferably capronic acid, urea, carbamate, sulfonamide or alkylamine.

8. A bioconjugate according to claim 4, **characterized in that** the linker attached on the amino acid Lysine is selected from NHS-ester, isocyanates, benzoyl fluorides or carbamates.

9. A bioconjugate according to any of the previous claims for use in the treatment and/or prevention of inflammatory and autoimmune ophthalmic diseases.

10. A bioconjugate or a polypeptide for use according to claim 9, **characterized in that** said inflammatory and autoimmune ophthalmic diseases are selected from keratoconjunctivitis sicca, vernal keratoconjunctivitis, stromal herpetic keratitis, corneal allograft rejection, corneal infections such as herpes and Pseudomonas aeruginosa keratitis or dry eye disease, preferably said autoimmune ophthalmic diseases is dry eye disease.

11. A bioconjugate or a polypeptide for use according to the peptide of formula (II) in the treatment of dry eye disease.

12. Pharmaceutical composition comprising at least one a bioconjugate or a polypeptide according to any of claims 1 to 8 and at least one pharmaceutically acceptable excipient.

13. Pharmaceutical composition according to claim 12, being formulated in topical administration.

14. A bioconjugate or a polypeptide for use according to claim 9, **characterized in that** it is administered as the only active principle or in combination with further active principles.

15. A bioconjugate or a polypeptide for use according to claim 14, **characterized in that** said further active principle is an adjuvant, immunesuppressive agent, immune modulating agent or inflammatory agent.
